(19) Europäisches Patentamt / European Patent Office / Office européen des brevets

(11) **EP 3 982 370 A1**

(12) **EUROPEAN PATENT APPLICATION**

(43) Date of publication:
**13.04.2022 Bulletin 2022/15**

(21) Application number: **21188468.9**

(22) Date of filing: **29.07.2021**

(51) International Patent Classification (IPC):
**G16C 20/40** $^{(2019.01)}$ **G16B 15/00** $^{(2019.01)}$

(52) Cooperative Patent Classification (CPC):
**G16C 20/40; G16B 15/00**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.10.2020 JP 2020170246**

(71) Applicant: **FUJITSU LIMITED**
**Kanagawa 211-8588 (JP)**

(72) Inventor: **Sato, Hiroyuki**
**Kawasaki-shi, Kanagawa, 211-8588 (JP)**

(74) Representative: **Hoffmann Eitle**
**Patent- und Rechtsanwälte PartmbB**
**Arabellastraße 30**
**81925 München (DE)**

(54) **STRUCTURE SEARCH PROGRAM, STRUCTURE SEARCH DEVICE, AND STRUCTURE SEARCH METHOD**

(57)    A structure search program configured to search for a structure of a compound in which a plurality of groups is linked, in which a computer performs a process of: determining an objective function including a constraint term which is a term for making a coefficient value to a predetermined value, the coefficient value expressing an inter-group distance with reference a shortest distance among distances between lattice points of a plurality of lattice points in a three-dimensional lattice space, the inter-group distance being a distance between a first group that is arranged at a first lattice point and a second group that is arranged at a second lattice point and is linked to the first group; and creating a three-dimensional structure of the compound by arranging the plurality of groups at lattice points that is a set of the plurality of lattice points based on the objective function.

**FIG. 14**

START

DEFINE THREE-DIMENSIONAL LATTICE SPACE — S101

DEFINE SET OF LATTICE POINTS AT WHICH i-TH AMINO ACID GROUPS ARE ARRANGED AS $V_i$ — S102

ALLOCATE BITS TO BE USED FOR CALCULATION TO EACH OF LATTICE POINTS — S103

DEFINE OBJECTIVE FUNCTION EQUATION REPRESENTED BY EQUATION (3) INCLUDING CONSTRAINT TERM REPRESENTED BY EQUATION (1) — S104

CONVERT OBJECTIVE FUNCTION EQUATION INTO ISING MODEL EQUATION OF EQUATION (4) — S105

MINIMIZE EQUATION (4) USING ANNEALING MACHINE — S106

SPECIFY THREE-DIMENSIONAL STRUCTURE OF PROTEIN ON THE BASIS OF STATE OF BIT THAT GIVES MINIMUM VALUE TO EQUATION (4) AND SPECIFY STABLE STRUCTURE OF PROTEIN — S107

OUTPUT STABLE STRUCTURE OF PROTEIN — S108

END

EP 3 982 370 A1

**Description**

[Field]

The present case relates to a structure search program, a structure search device, and a structure search method.

[Background Art]

In recent years, in situations such as drug discovery, there are some cases where a stable structure of a molecule having a relatively large size needs to be obtained using a computer. However, for example, there are some cases where a search for the stable structure within a realistic time is difficult in a calculation considering exposure of all of atoms for relatively large molecules in size such as peptides and proteins.

Therefore, a technology for shortening the calculation time by roughly grasping (coarse-graining) the structure of a molecule has been being researched. As a technology for coarse-graining a molecular structure, for example, a technology of coarse-graining a protein into a linear (continuous) simple cubic lattice structure on the basis of one-dimensional sequence information of amino acid residues in the protein, and treating the protein as a lattice protein has been researched. In the technology using a lattice protein, a technology for searching for a stable structure at high speed, using a quantum annealing technology, has been reported.

In the technology using a lattice protein, for example, the stable structure of the protein is searched using an objective function equation based on a plurality of constraints regarding arrangements of amino acid residues in the protein for which the stable structure is to be searched.

However, in the above-described objective function equation based on a plurality of constraints, satisfying the plurality of constraints at the same time is sometimes difficult, and the structure of the protein may not be able to be efficiently searched.

[Citation List]

[Non Patent Literature]

[NPL 1] R. Babbush et al., "Construction of Energy Functions for Lattice Heteropolymer Models: A Case Study in Constraint Satisfaction Programming and Adiabatic Quantum Optimization", Advances in Chemical Physics, 155, 201-244.

[Summary of Invention]

[Technical Problem]

In one aspect, an object of the present embodiment to provide a structure search program, a structure search device, and a structure search method capable of efficiently searching for a structure of a compound in which a plurality of groups is linked.

In one aspect of embodiment, a structure search program for searching for a structure of a compound in which a plurality of groups is linked, the program for causing a computer to perform a process of arranging the plurality of groups at lattice points in a three-dimensional lattice space that is a set of a plurality of lattice points based on an objective function including a constraint term which is a term for making a coefficient value to a predetermined value, the constraint term expressing an inter-group distance with reference a shortest distance among distances between lattice points of a plurality of lattice points in the three-dimensional lattice space, the inter-group distance being a distance between a first group among the plurality of groups, the first group being arranged at a first lattice point among the plurality of lattice points, and a second group that is one of the plurality of groups, the second group being arranged at a second lattice point among the plurality of lattice points, and is linked to the first group, and creating a three-dimensional structure of the compound in the three-dimensional lattice space by the arranging.

[Advantageous Effects of Invention]

In one aspect, the present embodiment can provide a structure search program, a structure search device, and a structure search method capable of efficiently searching for a structure of a compound in which a plurality of groups is linked.

[Brief Description of Drawings]

FIG. 1A is a schematic diagram illustrating an example of coarse-graining a protein and searching for a stable structure;

FIG. 1B is a schematic diagram illustrating an example of coarse-graining a protein and searching for a stable structure;

FIG. 1C is a schematic diagram illustrating an example of coarse-graining a protein and searching for a stable structure;

FIG. 2A is a schematic diagram for describing an example of a diamond encoding method;

FIG. 2B is a schematic diagram for describing an example of the diamond encoding method;

FIG. 2C is a schematic diagram for describing an example of the diamond encoding method;

FIG. 2D is a schematic diagram for describing an example of the diamond encoding method;

FIG. 2E is a schematic diagram for describing an example of the diamond encoding method;

FIG. 3 is a diagram for describing an example of $H_{one}$;

FIG. 4 is a diagram for describing an example of $H_{olap}$;

FIG. 5 is a diagram for describing an example of $H_{conn}$ in the prior art;

FIG. 6 is a diagram for describing an example of $H_{pair}$;

FIG. 7 is a diagram for describing another example of $H_{conn}$;

FIG. 8 is a diagram illustrating an example of a relationship between a function value and a variable of a function expressed by the equation (E);

FIG. 9 is a diagram illustrating an example of a relationship between a function value and a variable of a constraint term for causing a coefficient value for an inter-group distance expressed with reference to a shortest distance to become a predetermined value in an example of the technology disclosed in the present embodiment;

FIG. 10 is a diagram illustrating an example of a relationship between an inter-group distance and a shortest distance in a lattice space;

FIG. 11 is a diagram illustrating a hardware configuration example of a structure search device disclosed in the present embodiment;

FIG. 12 is a diagram illustrating another hardware configuration example of the structure search device disclosed in the present embodiment;

FIG. 13 is a diagram illustrating a functional configuration example of the structure search device disclosed in the present embodiment;

FIG. 14 is an example of a flowchart when searching for a stable structure of a protein using an example of the technology disclosed in the present embodiment;

FIG. 15 is a diagram illustrating an example in a case where each lattice with a radius r is $S_r$;

FIG. 16A is a diagram illustrating an example of a set of lattice points at which amino acid residues are arranged;

FIG. 16B is a diagram illustrating an example of a set of lattice points at which amino acid residues are arranged;

FIG. 16C is a diagram illustrating an example of a set of lattice points at which amino acid residues are arranged;

FIG. 16D is a diagram illustrating an example of a set of lattice points at which amino acid residues are arranged;

FIG. 17 is a diagram illustrating an example of a case where $S_1$, $S_2$, and $S_3$ are three-dimensionally illustrated;

FIG. 18A is a diagram illustrating an example of a state of allocating spatial information to bits $X_1$ to $X_n$;

FIG. 18B is a diagram illustrating an example of a state of allocating spatial information to bits $X_1$ to $X_n$;

FIG. 18C is a diagram illustrating an example of a state of allocating spatial information to bits $X_1$ to $X_n$;

FIG. 19 is a diagram for describing an example of $H_{one}$;

FIG. 20 is a diagram for describing an example of $H_{olap}$;

FIG. 21A is a diagram for describing an example of $H_{pair}$;

FIG. 21B is a diagram for describing an example of $H_{pair}$;

FIG. 22 is a diagram illustrating an example of a functional configuration of an annealing machine used for an annealing method;

FIG. 23 is a diagram illustrating an example of an operation flow of a transition control unit;

FIG. 24A is a diagram illustrating an example of an energy value and bit numbers of "1" for seven types on a low energy side in a case of setting parameters of $\lambda_{one}$, $\lambda_{olap}$, and $\lambda_{conn}$ to a same value that is an integer multiple of 5 from 5 to 30 in Comparative Example 1;

FIG. 24B is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 1;

FIG. 24C is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 1;

FIG. 24D is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 1;

FIG. 24E is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 1;

FIG. 24F is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 1;

FIG. 25A is a diagram illustrating an example of the energy value and the bit numbers of "1" for the seven types on the low energy side in a case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

FIG. 25B is a diagram illustrating an example of the energy value and the bit numbers of "1" for the seven types on the low energy side in the case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

FIG. 25C is a diagram illustrating an example of the energy value and the bit numbers of "1" for the seven types on the low energy side in the case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

FIG. 25D is a diagram illustrating an example of the energy value and the bit numbers of "1" for the seven types on the low energy side in the case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

FIG. 25E is a diagram illustrating an example of the energy value and the bit numbers of "1" for the seven types on the low energy side in the case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

FIG. 25F is a diagram illustrating an example of the energy value and the bit numbers of "1" for the seven types on the low energy side in the case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

FIG. 26A is a diagram illustrating an example of the energy value and the bit numbers of "1" for the seven types on the low energy side in a case of fixing $\lambda_{one}$ and $\lambda_{olap}$ to 25 and setting $\lambda_{conn}$ to an integer multiple of 5 from 5 to 25, among the parameters of $\lambda_{one}$, $\lambda_{olap}$, and $\lambda_{conn}$, in Comparative Example 1;

FIG. 26B is a diagram illustrating an example of the energy value and the bit numbers of "1" for the seven types on the low energy side in the case of fixing $\lambda_{one}$ and $\lambda_{olap}$ to 25 and setting $\lambda_{conn}$ to an integer multiple of 5 from 5 to 25, among the parameters of $\lambda_{one}$, $\lambda_{olap}$, and $\lambda_{conn}$, in Comparative Example 1;

FIG. 26C is a diagram illustrating an example of the energy value and the bit numbers of "1" for the seven types on the low energy side in the case of fixing $\lambda_{one}$ and $\lambda_{olap}$ to 25 and setting $\lambda_{conn}$ to an integer multiple of 5 from 5 to 25, among the parameters of $\lambda_{one}$, $\lambda_{olap}$, and $\lambda_{conn}$, in Comparative Example 1;

FIG. 26D is a diagram illustrating an example of the energy value and the bit numbers of "1" for the seven types on the low energy side in the case of fixing $\lambda_{one}$ and $\lambda_{olap}$ to 25 and setting $\lambda_{conn}$ to an integer multiple of 5 from 5 to 25, among the parameters of $\lambda_{one}$, $\lambda_{olap}$, and $\lambda_{conn}$, in Comparative Example 1;

FIG. 26E is a diagram illustrating an example of the energy value and the bit numbers of "1" for the seven types on the low energy side in the case of fixing $\lambda_{one}$ and $\lambda_{olap}$ to 25 and setting $\lambda_{conn}$ to an integer multiple of 5 from 5 to 25, among the parameters of $\lambda_{one}$, $\lambda_{olap}$, and $\lambda_{conn}$, in Comparative Example 1;

FIG. 27A is a diagram illustrating an example of the energy value and the bit numbers of "1" for the seven types on the low energy side in a case of setting parameters of $\lambda_{one}$, $\lambda_{olap}$, and $\lambda_{conn}$ to a same value that is an integer multiple of 5 from 5 to 30 in Comparative Example 2;

FIG. 27B is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 2;

FIG. 27C is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 2;

FIG. 27D is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 2;

FIG. 27E is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 2;

FIG. 27F is a diagram illustrating an example of the energy value and the bit numbers of "1" for the seven types on the low energy side in the case of setting the parameters of $\lambda_{one}$, $\lambda_{olap}$, and $\lambda_{conn}$ to the same value that is an integer multiple of 5 from 5 to 30 in Comparative Example 2;

FIG. 28A is a diagram illustrating an example of an energy value and bit numbers of "1" for twenty types on the low energy side in Comparative Example 2;

FIG. 28B is a diagram illustrating the most stable structure of "PLP-2" obtained in Comparative Example 2;

FIG. 29A is a diagram illustrating an example of an energy value and bit numbers of "1" for seven types on a low energy side in Example 1;

FIG. 29B is a diagram illustrating an example of a search result of a three-dimensional structure of "PLP-2" in Example 1; and

FIG. 29C is a diagram illustrating an example of a search result of a stable structure of "PLP-2" (a result of the energy value "-432") searched in Example 1 and a structure specified by NMR of a particular cyclic peptide superimposed each other.

[Description of Embodiments]

(Structure Search Program)

The technology disclosed in the present embodiment is based on the findings of the present inventors that, in prior art, when searching for a structure of a compound in which a plurality of groups is arranged at lattice points in a three-dimensional lattice space that is also a set of lattice points, and the plurality of groups is linked, the structure of the compound is not able to be efficiently searched. Therefore, prior to detailed description of the technology disclosed in the present embodiment, the problem of the prior art and the like will be described using a case where the compound for which the structure is to be searched is a protein as an example.

When searching for a stable structure of a protein (or peptide), the technology of coarse-graining amino acid residues forming the protein and treating the protein as a lattice protein can be used, as described above. Here, as one of technologies using the lattice protein, a method of obtaining a folded structure as the stable structure of the protein by a diamond encoding method will be described.

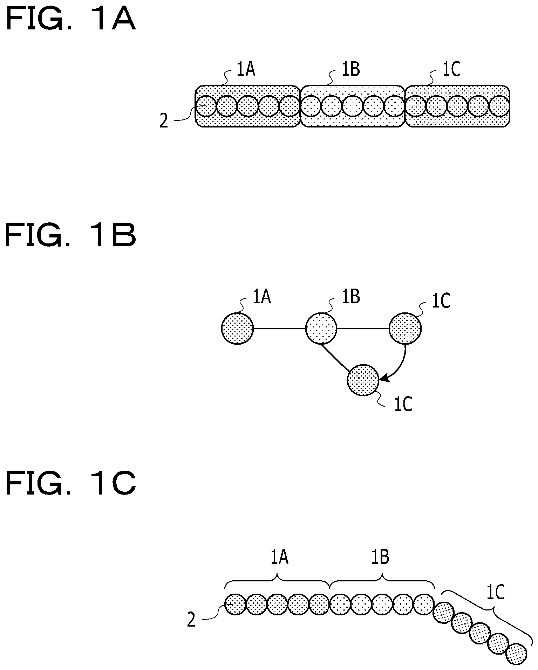

When searching for the structure of the protein (or peptide) using the lattice protein, first, the protein is coarse-grained. Here, the coarse-graining of the protein is performed by coarse-graining atoms 2 constituting the protein into coarse-grained particles 1A, 1B, and 1C that are units of each amino acid residue and creating a coarse-grained model, as illustrated in FIG. 1A, for example.

Next, a stable bonding structure is searched using the created coarse-grained model. FIG. 1B illustrates an example of a case where the bonding structure having the coarse-grained particle 1C located at an end point of the arrow is stable. Here, the search for the stable bonding structure is performed by the diamond encoding method to be described below.

Then, as illustrated in FIG. 1C, the coarse-grained model is returned to an all-atom model based on the stable bonding structure searched using the diamond encoding method.

Here, the diamond encoding method is usually a method of applying coarse-grained particles (coarse-grained model) of chain-shaped amino acids forming a protein to lattice points of a diamond lattice, and can express a three-dimensional protein structure.

Hereinafter, for simplification of the description, the diamond encoding method will be described by taking a case of a two-dimensional simple cubic lattice as an example.

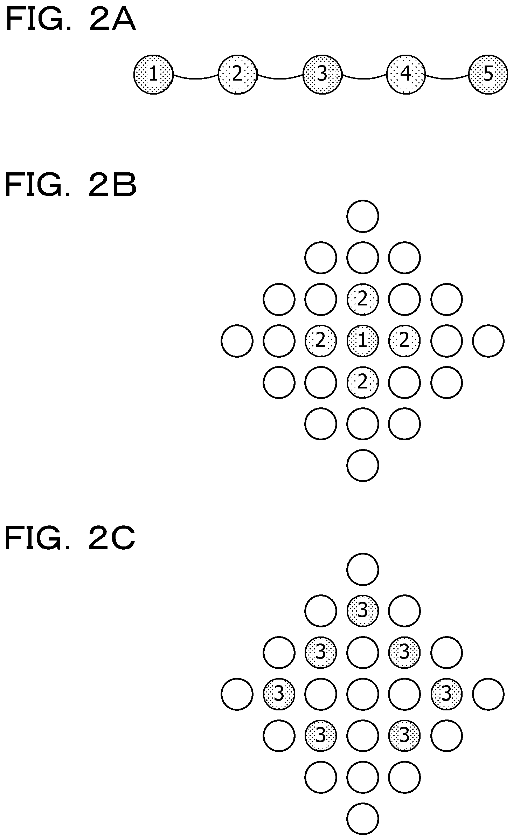

FIG. 2A is a diagram illustrating an example of a structure in a case where a linear pentapeptide having bonded five amino acid residues has a linear structure. Furthermore, in FIGs. 2A to 2E, the numbers in the circles represent numbers of the amino acid residues in the linear pentapeptide.

In the diamond encoding method, first, when the amino acid residue of the number 1 is arranged in the center of the diamond lattice, locations where the amino acid residue of the number 2 can be arranged, as illustrated in FIG. 2A, are limited to the locations illustrated in FIG. 2B (locations numbered 2) adjacent to the center. Next, locations where the amino acid residue of the number 3 bonded to the amino acid residue of the number 2 are limited to the locations (locations numbered 3) in FIG. 2C, which are adjacent to the locations numbered 2 in FIG. 2B.

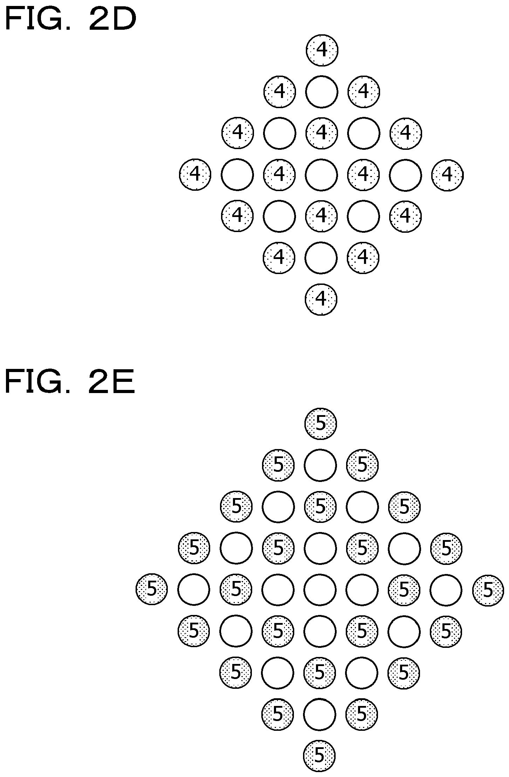

Then, locations where the amino acid residue of the number 4 bonded to the amino acid residue of the number 3 are limited to the locations (locations numbered 4) in FIG. 2D, which are adjacent to the locations numbered 3 in FIG. 2C. Moreover, locations where the amino acid residue of the number 5 bonded to the amino acid residue of the number 4 are limited to the locations (locations numbered 5) in FIG. 2E, which are adjacent to the locations numbered 4 in FIG. 2D.

By connecting the specified arrangeable places in the order of the numbers of the amino acid residues, the coarse-grained protein structure can be expressed.

The coarse-grained amino acid residues are arranged at the lattice points in the three-dimensional lattice space that is a set of lattice points by using the diamond encoding or the like in this way, a three-dimensional structure of the protein (peptide) can be created in the three-dimensional lattice space.

Here, when creating the three-dimensional structure of the protein (peptide) in the three-dimensional lattice space and searching for the structure of the protein, it is needed to appropriately select a combination of arrangements of the coarse-grained amino acid residues in the three-dimensional lattice space. To appropriately select a combination of arrangements of the coarse-grained amino acid residues, for example, it is favorable to determine the arrangements of the amino acid residues so that the arrangements of the amino acid residues satisfy a predetermined condition, for example.

The condition for the arrangements of the amino acid residues can be, for example, a condition that enables the three-dimensional structure created by arranging the amino acid residues in the three-dimensional lattice space to be a structure that can consistently exist as the protein (peptide), and an energetically stable structure. Such a condition can be, for example, a condition including the following three constraints and an interaction among the amino acid residues. [Constraints]

- The number of each of the amino acid residues forming the protein (peptide) is only one.
- The amino acid residues forming the protein (peptide) do not overlap with one another (the amino acid residues do not overlap at one lattice point).
- The amino acid residues forming the protein (peptide) are connected to one another (among the amino acid residues, the amino acid residues that are peptide-bonded to each other exist at adjacent lattice points in the three-dimensional lattice space) [interaction].
- There is an interaction between the amino acid residues that are not peptide-bonded to each other among the amino acid residues forming the protein (peptide).

That is, when creating the three-dimensional structure of the protein in the three-dimensional lattice space and searching for the stable structure of the protein, it is favorable to search for a structure that satisfies the above-described three constraints and in which the interaction between the amino acid residues that are not peptide-bonded to each other is stable (the energy is low).

Here, when searching for the structure that satisfies the above-described three constraints and in which the interaction between the amino acid residues that are not peptide-bonded to each other is stable, an objective function equation including the three constraints and the interaction as terms (functions) can be used, for example. For example, the stable structure of the protein can be searched by searching for an arrangement of the amino acid residues having the smallest value of such an objective function equation.

When including $H_{one}$, $H_{olap}$, and $H_{conn}$ as the constraint conditions and setting $H_{pair}$ as a term representing the interaction as such an objective function equation, for example, the objective function equation representing total energy in the diamond encoding method can be expressed by the following mathematical equation.

$$E(x) = H = H_{one} + H_{olap} + H_{conn} + H_{pair}$$

Here, $H_{one}$ represents the constraint that the number of each of the 1st to n-th amino acid residues is only one.

$H_{olap}$ represents the constraint that the 1st to n-th amino acid residues do not overlap with one another (the amino acid residues do not overlap at one lattice point).

$H_{conn}$ represents the constraint that the 1st to n-th amino acid residues are connected to one another (among the amino acid residues, the amino acid residues that are peptide-bonded to each other exist at adjacent lattice points in the three-dimensional lattice space).

$H_{pair}$ represents the interaction between the amino acid residues.

The following equation (A) is a mathematical equation representing a specific example of $H_{one}$ of the prior art in the above-described mathematical equation.

$$H_{one} \mathrel{+}= C_1 q_i q_j$$

Equation (A)

Here, $C_1$ is a coefficient for weighting and is a positive integer. $q_i$ takes "1" or "0". $q_j$ takes "1" or "0".

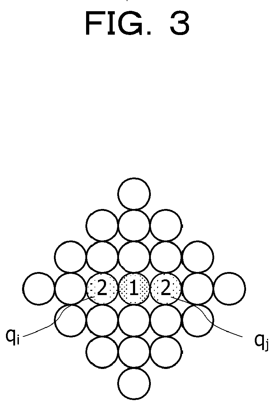

For the above-described $H_{one}$, as illustrated in FIG. 3, for example, in the case where two amino acid residues numbered 2 are present in the lattice space, both $q_i$ and $q_j$ are "1" (meaning that the amino acid residue is arranged). Therefore, $H_{one}$ represented by the above-described equation (A) has a positive value. Therefore, in the $H_{one}$ represented by the above-described equation (A), in the case where two identical amino acid residues are present, the $H_{one}$ becomes a positive value and increases the value of the objective function equation representing the total energy.

Therefore, by searching for an arrangement of the amino acid residues so that the value of $H_{one}$ expressed by the above-described equation (A) becomes smaller (for example, 0), the constraint that the number of each of the 1st to nth amino acid residues is only one can be implemented.

Next, the following equation (B) is a mathematical equation representing a specific example of the prior art of $H_{olap}$ in the above-described objective function equation representing the total energy.

$$H_{\mathrm{olap}} += C_2 q_i q_j \qquad \text{Equation (B)}$$

Here, $C_2$ is a coefficient for weighting and is a positive integer. $q_i$ takes "1" or "0". $q_j$ takes "1" or "0".

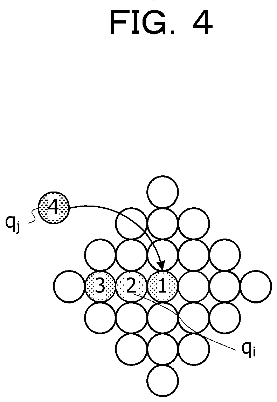

Regarding the above-described $H_{olap}$, as illustrated in FIG. 4, for example, in a case where the amino acid residue ($q_i$) numbered 2 and the amino acid residue ($q_j$) numbered 4 overlap at one lattice point, both the $q_i$ and $q_j$ are "1", so the $H_{olap}$ represented by the above-described equation (B) is a positive value. Therefore, in the $H_{olap}$ represented by the above-described equation (B), in the case where different amino acid residues are arranged overlapping with each other, the $H_{olap}$ becomes a positive value and increases the value of the objective function equation representing total energy.

Therefore, by searching for an arrangement of the amino acid residues so that the value of $H_{olap}$ expressed by the above-described equation (B) becomes smaller (for example, 0), the constraint that the 1st to nth amino acid residues do not overlap with one another can be implemented.

Next, the following equation (C) is a mathematical equation representing a specific example of the prior art of $H_{conn}$ in the above-described objective function equation representing the total energy.

$$H_{\mathrm{conn}} -= C_3 q_i q_j \qquad \text{Equation (C)}$$

Here, $C_3$ is a coefficient for weighting and is a positive integer. $q_i$ takes "1" or "0". $q_j$ takes "1" or "0".

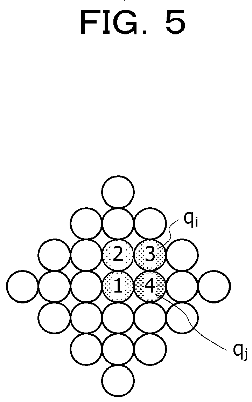

Regarding the above-described $H_{conn}$, first, consider the relationship between the amino acid residue ($q_i$) numbered 3 and the amino acid residue ($q_j$) numbered 4 that are the amino acid residues linked (adjacent) to each other in the protein for which the structure is to be searched, as illustrated in FIG. 5. At this time, in the case where the amino acid residue ($q_i$) numbered 3 and the amino acid residue ($q_j$) numbered 4 are arranged at positions adjacent to each other in the lattice space, both the $q_i$ and $q_j$ are "1", so the $H_{conn}$ represented by the above-described equation (C) becomes a negative value. Therefore, in the $H_{conn}$ represented by the above-described equation (C), in the case where the amino acid residues that are peptide-bonded to each other are arranged at adjacent lattice points in the lattice space, $H_{conn}$ becomes a negative value and decreases the value of the objective function equation representing the total energy.

Therefore, by searching for an arrangement of the amino acid residues so that the value of $H_{conn}$ represented by the above-described equation (C) becomes smaller (for example, becomes a larger negative number), the constraint that the 1st to nth amino acid residues are connected to one another can be implemented.

Next, the following equation (D) is a mathematical equation representing a specific example of the prior art of $H_{pair}$ in the above-described objective function equation representing the total energy.

$$H_{\mathrm{pair}} += E_{14} q_i q_j \qquad \text{Equation (D)}$$

Here, $E_{14}$ is a coefficient related to an interaction and is a positive integer. $q_i$ takes "1" or "0". $q_j$ takes "1" or "0". The coefficient $E_{14}$ regarding the interaction is defined for each combination of two amino acid residues, for example. The coefficient $E_{14}$ regarding the interaction can be determined by referring to the miyazawa-jernigan (MJ) matrix or the like, for example.

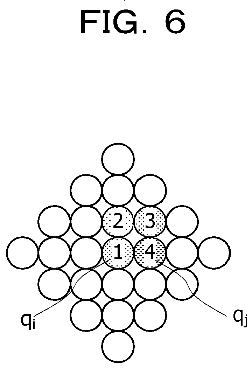

For the above-described $H_{pair}$, as illustrated in FIG. 6, for example, in the case where the amino acid residue ($q_i$) numbered 1 and the amino acid residue ($q_j$) numbered 4 are arranged adjacent to each other, the interaction between these amino acid residues can be expressed by the above-described equation (D).

Therefore, by searching for an arrangement of the amino acid residues so that the value of the $H_{pair}$ represented

by the above-described equation (D) becomes smaller (to have a more stable interaction), a more stable structure of the protein can be searched considering the interaction between the amino acid residues.

Here, as described above, in the case where the respective constraint is not satisfied, the $H_{one}$ represented by the above-described equation (A) and the $H_{olap}$ represented by the above-described equation (B) increases (destabilizes) the value of the objective function equation representing the total energy. That is, in the above-described prior art, the stable structure of the protein is searched using the $H_{one}$ destabilized when a plurality of the same amino acid residues exists, and the $H_{olap}$ destabilized when different amino acid residues are arranged overlapping with each other.

Furthermore, the $H_{conn}$ represented by the above-described equation (C) decreases (stabilizes) the value of the objective function equation representing the total energy when the constraint is satisfied. That is, in the above-described prior art, the stable structure of the protein is searched using the $H_{conn}$ stabilized when linked amino acid residues are arranged adjacent to each other on the basis of the relationship (relationship between two lattice points) established between the individual linked amino acid residues.

Here, the above-described $H_{one}$ represented by the above-described equation (A), the $H_{olap}$ represented by the above-described equation (B), and the $H_{conn}$ represented by the above-described equation (C) are not constraints independently of one another. Instead, when a certain constraint is satisfied, another constraint may be less likely to be satisfied. More specifically, in the prior art, the $H_{conn}$ contributing to stabilization and the $H_{one}$ and the $H_{olap}$ contributing to destabilization are competing (competitive), and it may be difficult to satisfy all the constraints at the same time, and the structure may not be able to be efficiently searched.

Furthermore, regarding the $H_{conn}$ representing the constraint that the amino acid residues in the protein are connected to one another, there is a technology using constraints based on the relationship between a certain lattice point and all the lattice points adjacent to the certain lattice point.

The constraints based on the relationship between a certain lattice point and all the lattice points adjacent to the certain lattice point can be, for example, constraints represented by (1) and (2) below.

(1) A constraint that, when the amino acid residue is present at a certain lattice point, the amino acid residue is present at only one lattice point among all the lattice points adjacent to the certain lattice point.

(2) A constraint that, when the amino acid residue is not present at a certain lattice point, no amino acid residue is present at all the lattice points adjacent to the certain lattice point or the amino acid residue is present at only one lattice point among all the lattice points adjacent to the certain lattice point.

This constraint can be represented by, for example, the following equations (E). Note that the equations (E) are an example when using the diamond encoding method of a two-dimensional case.

$$H \mathrel{+}= C(Q - q_0)(Q - 1)$$
$$Q = \Sigma_{i \in \eta(q_0)} q_i = q_1 + q_2 + q_3 + q_4 \qquad \text{Equation (E)}$$

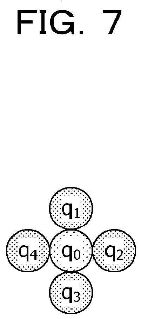

In the equations, C is a coefficient for weighting and is a positive integer. Each of $q_0$, $q_1$, $q_2$, $q_3$, and $q_4$ takes "1" or "0". The positional relationship among the $q_0$, $q_1$, $q_2$, $q_3$, and $q_4$ is the positional relationship illustrated in FIG. 7.

$\eta(q_0)$ is a set of bits representing the amino acid residue adjacent to and linked to $q_0$.

Here, the case where $q_0$ is "1" means that there is the amino acid residue at a certain lattice point. Then, the case where $q_0$ is "1", H becomes "0" only when Q is "1". In the case of the positional relationship illustrated in FIG. 7, the Q becomes "1" when $q_1 + q_2 + q_3 + q_4 = 1$. In other words, in the case of the positional relationship illustrated in FIG. 7, the Q becomes "1" when only one of the $q_1$, $q_2$, $q_3$, and $q_4$ becomes "1".

Therefore, the Q becomes "1" when the amino acid residue is present at only one lattice point among all the lattice points adjacent to a certain lattice point.

Furthermore, the case where $q_0$ is "0" is the case where no amino acid residue is present at a certain lattice point. Then, in the case where $q_0$ is "0", the H becomes "0" when the Q is "0" or when the Q is "1". In the case of the positional relationship illustrated in FIG. 7, the Q becomes "0" when $q_1 + q_2 + q_3 + q_4 = 0$ or 1. In other words, the Q becomes "0" when all the $q_1$, $q_2$, $q_3$, and $q_4$ is "0" or when only one of the $q_1$, $q_2$, $q_3$, and $q_4$ is "1". Therefore, the Q becomes "0" when no amino acid residue is present at all the lattice points adjacent to the certain lattice point or when the amino acid residue is present at only one lattice point among all the lattice points adjacent to the certain lattice point.

The above-described equation (E) is a constraint term related to linkage of n amino acid residues, and represents a constraint that the value of the objective function equation representing the total energy is increased when the constraint is not satisfied. Therefore, by using the above-described equation (E) as the constraint ($H_{conn}$) that the amino acid residues are connected to one another, $H_{one}$, $H_{olap}$, and $H_{conn}$ can be made independent of one another. Therefore, by using the above equation (E) as $H_{conn}$, competing (competition) among the $H_{one}$, $H_{olap}$, and $H_{conn}$ can be eliminated. Therefore, all the constraints becomes easily satisfied, and the structure that can consistently exists as the protein can be easily searched.

However, the H ($H_{conn}$) in the above-described equation (E) is a binary function determined by the $q_0$, $q_1$, $q_2$, $q_3$, and $q_4$ that take the value of "1" or "0", and is a function having a flat function shape.

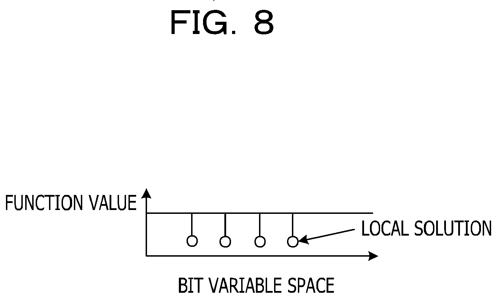

FIG. 8 is a diagram illustrating an example of a relationship between a function value and a variable of the function (constraint term) represented by the equation (E). As illustrated in FIG. 8, the above-described equation (E) is a function with a constant value except that local solutions with low function values are present in places in a bit variable space that the $q_0$, $q_1$, $q_2$, $q_3$, and $q_4$ can take, and has a flat function shape (a binary function value with no peaks or valleys). Therefore, for example, even if one local solution is reached, there is no index (clue) for searching for and reaching another local solution, the structure search becomes inefficient, and the search for a stable structure has been sometimes difficult.

As described by taking the case where the compound is the protein and the amino acid residues are arranged at the lattice points as an example, in the prior art, the constraint regarding the linked state of a plurality of groups in the objective function equation is not independent of the other constraints, and in some cases, it has been difficult to satisfy all the constraints at the same time. Furthermore, in another technology, the function shape of the constraint term representing the constraint regarding the linked state of a plurality of groups in the objective function equation is flat, and the structure search has been sometimes inefficient.

As described above, these technologies have not been able to efficiently search for a structure of a compound in which a plurality of groups is linked.

Therefore, the present inventors have made extensive studies on a program and the like capable of efficiently searching for a structure of a compound in which a plurality of groups is linked and have obtained the following findings.

That is, the present inventors have found that a structure of a compound in which a plurality of groups is linked can be efficiently searched by a following structure search program and the like.

The structure search program as an example of the technology disclosed in the present embodiment is a structure search program for searching for a structure of a compound in which a plurality of groups is linked, the program for causing a computer to perform a process of arranging the plurality of groups at lattice points in a three-dimensional lattice space that is a set of a plurality of lattice points based on an objective function equation including a constraint term which is a term for making a coefficient value to a predetermined value, the constraint term expressing an inter-group distance with reference a shortest distance among distances between lattice points of a plurality of lattice points in the three-dimensional lattice space, the inter-group distance being a distance between a first group among the plurality of groups, the first group being arranged at a first lattice point among the plurality of lattice points, and a second group that is one of the plurality of groups, the second group being arranged at a second lattice point among the plurality of lattice points, and is linked to the first group, and creating a three-dimensional structure of the compound in the three-dimensional lattice space by the arranging.

Here, in an example of the technology disclosed in the present embodiment, when searching for a structure of a compound in which a plurality of groups is linked, the plurality of groups is arranged at lattice points in a three-dimensional lattice space as a set of a plurality of lattice points, and a three-dimensional structure of the compound is created in the three-dimensional lattice space.

In an example of the technology disclosed in the present embodiment, when arranging the plurality of groups at the lattice points, the distance between groups to be linked to each other (inter-group distance) is expressed by a coefficient value with reference to the shortest distance (shortest distance between lattice points) among distances between lattice points of the plurality of lattice points. Then, in an example of the technology disclosed in the present embodiment, the plurality of groups is arranged at the lattice points on the basis of an objective function equation including a constraint term that causes the above-described coefficient value to become a predetermined value.

Here, the constraint term that causes the coefficient value to become a predetermined value is a constraint term regarding a linked state between a first group among the plurality of groups, the first group being arranged at a first lattice point among the plurality of lattice points, and a second group that is one of the plurality of groups, the second group being arranged at a second lattice point among the plurality of lattice points, and is linked to the first group, in the three-dimensional lattice space, for example. That is, the constraint term for causing the coefficient value to become a predetermined value can be, for example, a constraint term representing the constraint ($H_{conn}$) that a plurality of groups is connected to one another in the compound for which the structure is to be searched.

In the constraint term for causing the coefficient value to become a predetermined value, there is no particular limitation on arrangements of the first group (one group) and the second group (the other group) arranged in the three-dimensional lattice space, and the coefficient value can be expressed using the inter-group distance of a case of arranging the first and second groups at arbitrary lattice points. Therefore, the constraint term for causing the coefficient value to become a predetermined value can consider, for the lattice points in the three-dimensional lattice space in which the plurality of groups is arranged, not only the relationship between adjacent lattice points but also the relationship among all the lattice points (among all the prepared bits) existing in the three-dimensional lattice space.

Therefore, in an example of the technology disclosed in the present embodiment, the constraints on the structure of the compound contained in the objective function equation (for example, $H_{one}$, $H_{olap}$, and $H_{conn}$) can be made independent of one another. Therefore, in an example of the technology disclosed in the present embodiment, the competing (competition) between the constraints for the structure of the compound contained in the objective function equation can be eliminated. Therefore, all the constraints become easily satisfied, and the structure that can consistently exists as a compound can be easily searched.

Furthermore, the coefficient value for the inter-group distance expressed with reference to the shortest distance can be a coefficient value corresponding to the magnitude of the inter-group distance with respect to the shortest distance, for example. Therefore, the coefficient value for the inter-group distance expressed with reference to the shortest distance can be a coefficient value, for example, that becomes large when the inter-group distance is large (long) and becomes small when the inter-group distance is small (short). Moreover, the coefficient value for the inter-group distance expressed with reference to the shortest distance can be a coefficient value that takes the minimum value when the inter-group distance matches the shortest distance (when the inter-group distance becomes the shortest), for example.

Then, the constraint term for causing the coefficient value to become a predetermined value can be a constraint term that constrains the coefficient value to become small, for example. That is, the constraint term for causing the coefficient value to become a predetermined value can be a constraint term for causing the inter-group distance between groups linked to each other and the shortest distance between lattice points to become close to each other.

In this way, in an example of the technology disclosed in the present embodiment, the constraint term for causing the coefficient value to become a predetermined value can be, for example, a constraint term for making the coefficient value for the inter-group distance expressed with reference to the shortest distance small (for making the inter-group distance and the shortest distance close to each other). More specifically, the constraint term for causing the coefficient value to become a predetermined value is favorably a constraint term, for example, for constraining the inter-group distance and the shortest distance to match to make the coefficient value approach "0" (to make the predetermined value "0"). By doing so, the stable structure of the compound can be more reliably created (searched).

In an example of the technology disclosed in the present embodiment, by using the constraint term for causing the coefficient value to become a predetermined value, as described above, a constraint term representing the constraint ($H_{conn}$) that a plurality of groups is connected to one another in the compound for which the structure is searched can be represented, for example.

The constraint term for causing the coefficient value to become a predetermined value as described above is not particularly limited, and can be appropriately selected according to the purpose. Examples of the constraint term for causing the coefficient value to become a predetermined value include a constraint term expressing the coefficient value using a difference between the inter-group distance and the shortest distance, a constraint term expressing the coefficient value using a ratio of the inter-group distance and the shortest distance, a constraint term expressing the coefficient value using a square of the difference between the inter-group distance and the shortest distance, and the like.

Here, in the coefficient value for the inter-group distance expressed with reference to the shortest distance, a multi-valued coefficient according to the inter-group distance can be adopted for the coefficient value corresponding to the magnitude of the inter-group distance with respect to the shortest distance, as described above. Therefore, by using the coefficient value for the inter-group distance expressed with reference to the shortest distance, the constraint term can be made into a function shape with an inclination (with peaks and valleys).

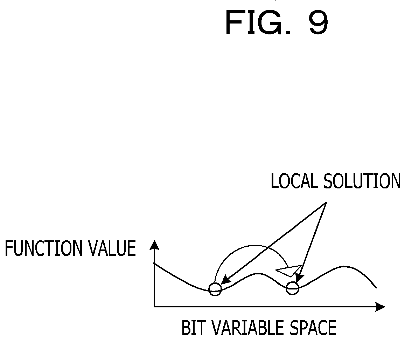

FIG. 9 is a diagram illustrating an example of the relationship between the function value and the variable of the constraint term for causing the coefficient value for the inter-group distance expressed with reference to the shortest distance to become a predetermined value, in an example of the technology disclosed in the present embodiment. As illustrated in FIG. 9, in the constraint term for causing the coefficient value to become a predetermined value in an example of the technology disclosed in the present embodiment, the function value can be formed into a function shape with an inclination (with peaks and valleys) in the bit variable space that the bits representing the lattice points can take. Therefore, in an example of the technology disclosed in the present embodiment, in a case where one local solution has been reached (for example, the local solution on the left side in FIG. 9), another local solution can be searched and the structure of the compound can be efficiently searched in consideration of the inclination (slope) of the surroundings.

As described above, in an example of the technology disclosed in the present embodiment, the plurality of groups is arranged at the lattice points based on the objective function equation including the constraint term that causes the above-described coefficient value to become a predetermined value, and the three-dimensional structure of the compound is created in the three-dimensional lattice space. Therefore, since an example of the technology disclosed in the present embodiment uses the objective function equation including the independent constraint term using the function shape with an inclination, the structure of the compound in which a plurality of groups is linked can be efficiently searched.

Hereinafter, in an example of the structure search program disclosed in the present embodiment, each process performed by a computer will be described in detail.

The structure search program disclosed in the present embodiment causes a computer to perform at least a process of creating a three-dimensional structure and further causes the computer to perform other steps as needed, for example.

The structure search program disclosed in the present embodiment can be created using various known programming languages according to the configuration of a computer system to be used, the type and version of an operating system, and the like.

The structure search program disclosed in the present embodiment may be recorded on a recording medium such as a built-in hard disk or an external hard disk, or may be recorded on a recording medium such as a compact disc read only memory (CD-ROM), a digital versatile disk read only memory (DVD-ROM), a magneto-optical (MO) disk, or a universal serial bus (USB) memory [USB flash drive], for example.

Moreover, in a case of recording the structure search program disclosed in the present embodiment in the above-described recording medium, the program can be directly used or can be installed into a hard disk and then used through a recording medium readout device included in the computer system, as needed. Furthermore, the structure search program disclosed in the present embodiment may be recorded in an external storage region (another computer or the like) accessible from the computer system through an information communication network. In this case, the structure search program disclosed in the present embodiment, which is recorded in the external storage region, can be used directly or can be installed in a hard disk and then used from the external storage region through the information communication network, as needed.

Note that the structure search program disclosed in the present embodiment may be divided for each of any pieces of processing and recorded in a plurality of recording media.

First, the structure search program disclosed in the present embodiment is a program for searching for a structure of a compound in which a plurality of groups is linked.

The compound for which the structure is to be searched is not particularly limited as long as the compound is a compound in which a plurality of groups (compound residues) is linked, and can be appropriately selected according to the purpose.

The plurality of groups is not particularly limited as long as the groups can be bonded to one another, and can be appropriately selected according to the purpose. Examples of the plurality of groups include amino acid residues, reactive monomers, and the like. In the case where the plurality of groups is the amino acid residues, for example, the compound can be a protein or a peptide. In the case where the plurality of groups is the reactive monomers, the compound can be a polymer. Among these examples, in an example of the technology disclosed in the present embodiment, the compound is favorably the protein or peptide, and the plurality of groups is favorably the amino acid residues. Note that, in an example of the technology disclosed in the present embodiment, for example, a compound in which a relatively large number of amino acid residues is linked may be called a protein, and a compound in which a relatively small number of amino acid residues is linked may be called a peptide.

Furthermore, the compound in which a plurality of groups is linked is not limited to a linear (continuous) compound and may have a branched structure in the compound.

An amino acid that is a source of the amino acid residue may be a natural amino acid or an unnatural amino acid (modified amino acid or artificial amino acid). Examples of the natural amino acid include alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, β-alanine, β-phenylalanine, and the like. Note that the number of amino acid residues in the peptide (protein) is not particularly limited and may be appropriately selected depending on the purpose, and may be, for example, about 10 or more and 50 or less, or several hundreds.

Furthermore, an example of the modified amino acid includes an amino acid obtained by modifying (substituting) a part of the structure of the natural amino acid as described above, or the like. Specifically, as the modified amino acid, for example, an amino acid or the like obtained by methylating a part of the structure of the natural amino acid can be used.

Furthermore, for example, in the case of arranging the amino acid residues at the lattice points, each amino acid residue may be treated as one particle, or the amino acid residues may be divided into a main chain and a side chain in the peptide (protein) and may be treated as different particles (a main chain particle and a side chain particle). In the case of dividing the amino acid residues into the main chain and the side chain in the peptide (protein) and treating the respective amino acid residue as separate particles, an amino acid that does not have a side chain (for example, glycine or the like) is favorably treated as a particle that can be a main chain particle and is also a side chain particle.

<Process of Creating Three-dimensional Structure (Three-dimensional Structure Creation Process)>

In the process of creating a three-dimensional structure (three-dimensional structure creation process), the plurality of groups is arranged at the lattice points in the three-dimensional lattice space that is a set of the plurality of lattice points, and the three-dimensional structure of the compound is created in the three-dimensional lattice space.

The type of the three-dimensional lattice space is not particularly limited and can be appropriately selected according to the purpose. Examples thereof include a simple cubic lattice, a body-centered cubic lattice, a face-centered cubic lattice, and the like.

Furthermore, in the process of creating a three-dimensional structure, for example, the inter-group distance between the first group (one group) among the plurality of groups, the first group being arranged at the first lattice point among the plurality of lattice points, and the second group (the other group) that is one of the plurality of groups, the second group being arranged at the second lattice point among the plurality of lattice points, and is linked to the first group, in the three-dimensional lattice space, is obtained. In other words, in the process of creating a three-dimensional structure, for example, the first group (one group) in the plurality of groups arranged in the three-dimensional lattice space is specified, and the inter-group distance to the second group (the other group) linked to the first group in the compound for which the structure is to be searched is obtained.

The technique of obtaining the inter-group distance is not particularly limited and can be appropriately selected according to the purpose. An example of the technique of obtaining the inter-group distance includes, for example, a technique of obtaining the distance between the first lattice point at which the first group is arranged and the second lattice point at which the second group is arranged on the basis of information of the positions of the lattice points in the three-dimensional lattice space in which the plurality of groups is arranged.

Then, in the process of creating a three-dimensional structure, the inter-group distance is expressed by the coefficient value with reference to the shortest distance between lattice points, and the plurality of groups is arranged at the lattice points on the basis of the objective function equation including the constraint term that causes the coefficient value to become a predetermined value.

<<Constraint Term For Causing Coefficient Value to Become Predetermined Value>>

As described above, the constraint term for causing the coefficient value to become a predetermined value is, for example, a constraint term regarding the linked state between the first group in the plurality of groups arranged in the three-dimensional lattice space and the second group linked to the first group in the compound for which the structure is to be searched. That is, the constraint term for causing the coefficient value to become a predetermined value can be, for example, a constraint term representing the constraint ($H_{conn}$) that a plurality of groups is connected to one another in the compound for which the structure is to be searched.

Here, the coefficient value for the inter-group distance expressed with reference to the shortest distance is not particularly limited as long as the relationship of the inter-group distance with respect to the shortest distance can be expressed, and can be appropriately selected according to the purpose.

The coefficient value for the inter-group distance expressed with reference to the shortest distance is favorably, for example, a coefficient value that becomes large when the inter-group distance is large (long) and becomes small when the inter-group distance is small (short), as described above. Moreover, the coefficient value for the inter-group distance expressed with reference to the shortest distance is favorably a coefficient value that takes the minimum value when the inter-group distance matches the shortest distance, for example.

Then, the constraint term for causing the coefficient value to become a predetermined value can be, for example, a constraint term for making the coefficient value for the inter-group distance expressed with reference to the shortest distance small (for making the inter-group distance and the shortest distance close to each other). More specifically, the constraint term for causing the coefficient value to become a predetermined value is favorably, for example, a constraint term for constraining the inter-group distance and the shortest distance to match to make the coefficient value approach "0". By doing so, the stable structure of the compound can be more reliably created (searched).

Examples of such a constraint term include, for example, a constraint term expressing the coefficient value using a difference between the inter-group distance and the shortest distance, a constraint term expressing the coefficient value using a ratio of the inter-group distance and the shortest distance, and a constraint term expressing the coefficient value using a square of the difference between the inter-group distance and the shortest distance.

As the constraint term expressing the coefficient value using the difference between the inter-group distance and the shortest distance, for example, the constraint term represented by the following equation (1) can be used.

$$H_{conn} = \sum_{n} \left[ \sum_{i \in a(n), j \in a(n+1)} \{abs(d_{ij}-d_0)q_i q_j\} \right] \quad \text{... Equation (1)}$$

Note that, in the equation (1), $H_{conn}$ is the constraint term for causing the coefficient value to be a predetermined value, $a(n)$ is a set of bit numbers in the n-th group, $a(n+1)$ is a set of bit numbers in the (n + 1)-th group, $d_{ij}$ is the inter-group distance between a group arranged at an i-th lattice point of the plurality of lattice points and a group arranged at a j-th lattice point of the plurality of lattice points, $d_0$ is the shortest distance, $abs(d_{ij} - d_0)$ is the coefficient value represented by an absolute value of a difference between $d_{ij}$ and $d_0$, $q_i$ is a binary variable of 0 or 1 that represents the presence or absence of the group arranged at the i-th lattice point, and $q_j$ is a binary variable of 0 or 1 that represents the presence or absence of the group arranged at the j-th lattice point.

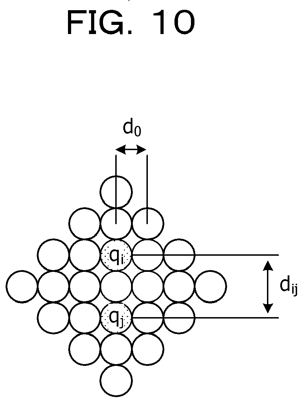

The above-described equation (1) will be described with reference to FIG. 10.

FIG. 10 is a diagram illustrating an example of the relationship between the inter-group distance and the shortest distance in the lattice space. In FIG. 10, it is assumed, for example, that the lattice point represented by $q_i$ is the first lattice point (one lattice point) at which the first group (one group) in the plurality of groups is arranged, and the lattice point represented by $q_j$ is the second lattice point (the other lattice point) that is an arrangement candidate for the second group (the other group) linked to the first group in the compound for which the structure is to be searched.

At this time, as illustrated in FIG. 10, the inter-group distance between the first group arranged at the lattice point represented by $q_i$ and the second group arranged at the lattice point represented by $q_j$ is $d_{ij}$. Moreover, as illustrated in FIG. 10, the shortest distance between lattice points is represented by do, which is the distance between lattice points located adjacent to each other.

In the above-described equation (1), the absolute value of the difference between the inter-group distance $d_{ij}$ and the shortest distance do between lattice points as in the relationship illustrated in FIG. 10 is used as the coefficient value. In the above-described equation (1), when the difference between the inter-group distance $d_{ij}$ and the shortest distance do becomes "0" (the inter-group distance $d_{ij}$ and the shortest distance do match), the coefficient value also becomes "0".

Therefore, in the constraint term represented by the above-described equation (1), when each coefficient value of when a plurality of groups is arranged becomes "0", the value of the constraint term also becomes "0 (minimum value)". Therefore, in the constraint term represented by the above-described equation (1), by searching for a combination of arrangements in which the value of the constraint term approaches "0", the constraint that the plurality of groups is linked to each other can be represented in the compound for which the structure is to be searched.

Furthermore, as the constraint term expressing the coefficient value using the ratio of the inter-group distance and the shortest distance, for example, the constraint term represented by the following equation (2) can be used.

$$H_{conn} = \sum_n \left[ \sum_{i \in a(n), j \in a(n+1)} \{abs\{(d_{ij}/d_0)-1\}q_i q_j\} \right] \quad \text{... Equation (2)}$$

Note that, in the equation (2), $H_{conn}$ is the constraint term for causing the coefficient value to be a predetermined value, $a(n)$ is a set of bit numbers in the n-th group of the plurality of groups, $a(n + 1)$ is a set of bit numbers in the (n + 1)-th group of the plurality of groups, $d_{ij}$ is the inter-group distance between the group arranged at the i-th lattice point and the group arranged at the j-th lattice point, $d_0$ is the shortest distance, $abs\{(d_{ij}/d_0) - 1\}$ is the coefficient value represented by an absolute value of a number obtained by subtracting 1 from the ratio of $d_{ij}$ and $d_0$, $q_i$ is the binary variable of 0 or 1 that represents the presence or absence of the group arranged at the i-th lattice point, and $q_j$ is the binary variable of 0 or 1 that represents the presence or absence of the group arranged at the j-th lattice point.

In the above-described equation (2), the absolute value of the number obtained by subtracting 1 from the ratio of the inter-group distance $d_{ij}$ and the shortest distance $d_0$ between lattice points as in the relationship illustrated in FIG. 10 is used as the coefficient value. In the above-described equation (2), when the difference between the inter-group distance dij and the shortest distance $d_0$ becomes "0" (the inter-group distance dij and the shortest distance $d_0$ match), the coefficient value also becomes "0".

Therefore, in the constraint term represented by the above-described equation (2), when each coefficient value of when a plurality of groups is arranged becomes "0", the value of the constraint term also becomes "0 (minimum value)". Therefore, in the constraint term represented by the above-described equation (2), by searching for a combination of arrangements in which the value of the constraint term approaches "0", the constraint that the plurality of groups is linked to each other can be represented in the compound for which the structure is to be searched.

Note that, in the technology disclosed in the present embodiment, it is not essential to search for the structure in which the coefficient value in the constraint term becomes "0" (for example, the most stable structure), a slightly unstable structure may be searched as long as the structure can exist as a compound, for example. Furthermore, even in the case of searching for the most stable structure in a compound, it is not essential to search for the structure in which the coefficient value in the constraint term becomes "0", and a structure in which the coefficient value becomes relatively small may be searched in consideration of the balance with parameters of other constraint terms.

<<Objective Function Equation>>

The objective function equation usually means a function based on conditions or constraints in a combination optimization problem, and is a function that takes the minimum value when variables (parameters) of the objective function equation have an optimum combination in the combination optimization problem. Note that the objective function equation (objective function) may also be referred to as an energy function, a cost function, Hamiltonian, or the like.

Here, arranging the plurality of groups (compound residues) at the lattice points in the three-dimensional lattice space and creating the three-dimensional structure of the compound in the three-dimensional lattice space can be considered to be an optimization problem of optimizing the combination of the compound residues to be arranged at the lattice points. Therefore, for example, by searching for the combination of variables in which the objective function equation has the minimum value, the solution of the combination optimization problem can be searched, that is, the stable three-dimensional structure of the compound can be searched in the three-dimensional lattice space.

The objective function equation is not particularly limited as long as the constraint term for causing the coefficient value to become a predetermined value is included and becomes a low value when the compound has a stable three-dimensional structure, and can be appropriately selected according to the purpose.

The objective function equation favorably includes, for example, at least the following four terms:

- a constraint term representing the constraint that the number of each of the plurality of groups is only one;
- a constraint term representing the constraint that the plurality of groups does not overlap with one another;
- a constraint term representing the constraint that the plurality of groups is connected to one another; and
- a term representing the interaction between the plurality of groups.

Here, the three terms other than the term representing the interaction between the plurality of groups among the above-described four terms can be considered as, for example, the constraint terms for causing the three-dimensional structure of the compound to be created to be the structure that can consistently exist as the compound. These three constraint terms can be, for example, terms in which the value becomes small (for example, the value becomes zero) when the constraint represented by each term is satisfied. By doing so, in an example of the technology disclosed in the present embodiment, since the value of the objective function equation becomes small when the searched three-dimensional structure of the compound is a structure that can consistently exist as the compound, for example, a more appropriate three-dimensional structure can be searched.

Furthermore, the above-described term representing the interaction between the plurality of groups can be considered as a term representing an interaction for causing the three-dimensional structure of the compound to be created to be an energetically stable structure. The term representing the interaction between the plurality of groups can be a term that takes a smaller value when the interaction is stable (the energy is low) according to the distance between the plurality of groups arranged at the lattice points in the three-dimensional lattice space, for example. By doing so, in an example of the technology disclosed in the present embodiment, since the value of the objective function equation becomes small when the searched three-dimensional structure of the compound is an energetically more stable structure, for example, a more appropriate three-dimensional structure can be searched.

That is, in an example of the technology disclosed in the present embodiment, the three-dimensional structure of the compound is created on the basis of the objective function equation including the above-described four terms, whereby the three-dimensional structure to be searched can be made the structure that can consistently exist as the compound and the energetically stable structure.

Furthermore, in an example of the technology disclosed in the present embodiment, as the objective function equation, the one expressed by the following equation (3) is favorably used. In an example of the technology disclosed in the present embodiment, by creating the three-dimensional structure of the compound by minimizing (optimizing) the following equation (3), for example, a more stable structure of the compound can be searched.

$$H_{total} = \{\lambda_{one} \times H_{one} + \lambda_{olap} \times H_{olap} + \lambda_{conn} \times (H_{conn} + C)\} + H_{pair} \quad \text{... Equation (3)}$$

Note that, in the equation (3), $H_{total}$ is the objective function equation, $H_{one}$ is the constraint term representing the constraint that the number of each of the plurality of groups is only one, $\lambda_{one}$ is the parameter for weighting the $H_{one}$, $H_{olap}$ is the constraint term representing the constraint that the plurality of groups does not overlap with one another, $\lambda_{olap}$ is the parameter for weighting the $H_{olap}$, $H_{conn}$ represents the constraint that the plurality of groups is connected to one another, and is the constraint term represented by the equation (1) or (2), C is the constant term regarding the constraint that the plurality of groups is connected to one another, $\lambda_{conn}$ is the parameter for weighting the $H_{conn}$ and the C, and $H_{pair}$ is the term representing the interaction between the plurality of groups.

In the above-described equation (3), $H_{one}$, $H_{olap}$, and $H_{conn}$ are, for example, constraint terms for making a three-dimensional structure of a compound to be created a structure that can consistently exist as a compound, and can be terms having a small value (for example, a value of zero) when the constraint represented by each term is satisfied).

Furthermore, in the above-described equation (3), $H_{pair}$ is, for example, a term representing the interaction for causing the three-dimensional structure of the compound to be created to be the energetically stable structure, and can be a term having a smaller value when the interaction is stable (low energy).

Note that more specific expressions or the like of $H_{one}$, $H_{olap}$, $H_{conn}$, and $H_{pair}$ in the above-described equation (3) will be described below.

In the above-described equation (3), for example, the most stable structure of the compound can be searched by appropriately adjusting the parameters of $\lambda_{one}$, $\lambda_{olap}$, and $\lambda_{conn}$. Furthermore, when searching for the structure of the compound using the above-described equation (3), for example, calculations in which the parameters of $\lambda_{one}$, $\lambda_{olap}$, and $\lambda_{conn}$ are set to different values may be performed simultaneously in parallel.

Note that the parameters of $\lambda_{one}$, $\lambda_{olap}$, and $\lambda_{conn}$ can be, for example, positive integers.

Here, as the technique of minimizing the objective function equation is not particularly limited and can be appropriately selected according to the purpose. For example, a technique of minimizing the objective function equation on the basis of the objective function equation converted into an Ising model equation represented by the following equation (4) is favorable. In other words, in an example of the technology disclosed in this case, it is favorable to perform the processing of creating a three-dimensional structure by optimization processing based on the objective function equation converted in to the Ising model equation represented by the following equation (4). Note that the Ising model equation represented by the following equation (4) is an Ising model equation in a quadratic unconstrained binary optimization (QUBO) format.

$$E = -\sum_{i,j=0} w_{ij}\, x_i x_j - \sum_{i=0} b_i x_i$$

... Equation (4)

Note that, in the above-described equation (4), E is the objective function equation converted into the Ising model equation.

$w_{ij}$ is a numerical value representing the interaction between the i-th bit and the j-th bit.

$b_i$ is a numerical value representing the bias for the i-th bit.

$x_i$ is a binary variable representing that the i-th bit is 0 or 1.

$x_j$ is a binary variable representing that the j-th bit is 0 or 1.

Here, $w_{ij}$ in the above-described equation (4) can be obtained by, for example, extracting the numerical value or the like of each parameter in the objective function equation before being converted into the Ising model equation for each combination of $x_i$ and $x_j$, and is usually a matrix.

The first term on the right side in the above-described equation (4) is obtained by integrating the product of the state (state) and weight value (weight) of two circuits for all of combinations of two bits selectable from all the bits without omission or duplication.

Furthermore, the second term on the right side in the above-described equation (4) is obtained by integrating the product of the value and state of the bias of each of all the bits.

For example, by extracting the parameters of the objective function equation before being converted into the Ising model equation and obtaining $w_{ij}$ and bi, the objective function equation can be converted into the Ising model equation expressed by the above-described equation (4).

The objective function equation converted into the Ising model equation as described above can be optimized (minimized) in a short time by, for example, performing the annealing method (annealing) using the annealing machine or the like. That is, in an example of the technology disclosed in the present embodiment, the process of creating a three-dimensional structure is favorably performed by calculating the minimum energy in the Ising model equation by executing the ground state search using the annealing method for the Ising model equation.

The annealing machine used to optimize the objective function equation is, for example, a quantum annealing machine, a semiconductor annealing machine using the semiconductor technology, a machine of performing simulated annealing (simulated annealing) executed by software by using a central processing unit (CPU) or a graphics processing unit (GPU), or the like. Furthermore, for example, Digital Annealer (registered trademark) may be used as the annealing machine.

Note that details of the annealing method using the annealing machine will be described below.

<Other Processes>

Other processes are not particularly limited and can be appropriately selected according to the purpose.

(Structure Search Method)

The structure search method disclosed in the present embodiment is a structure search method for searching for a stable structure of a compound in which a plurality of groups is linked, the method including a process of arranging the plurality of groups at lattice points in a three-dimensional lattice space that is a set of a plurality of lattice points based on an objective function equation including a constraint term which is a term for making a coefficient value to a predetermined value, the constraint term expressing an inter-group distance with reference a shortest distance among distances between lattice points of a plurality of lattice points in the three-dimensional lattice space, the inter-group distance being a distance between a first group among the plurality of groups, the first group being arranged at a first lattice point among the plurality of lattice points, and a second group that is one of the plurality of groups, the second group being arranged at a second lattice point among the plurality of lattice points, and is linked to the first group, and creating a three-dimensional structure of the compound in the three-dimensional lattice space by the arranging.

The structure search method disclosed in the present embodiment can be performed similarly to the process of creating a three-dimensional structure in the structure search program disclosed in the present embodiment, for example. Furthermore, a favorable mode in the structure search method disclosed in the present embodiment can be made similar to the favorable mode of the process of creating a three-dimensional structure in the structure search program disclosed in the present embodiment, for example.

The structure search method disclosed in the present embodiment can be, for example, a method of performing the process of creating a three-dimensional structure using a computer.

(Structure Search Device)

The structure search device disclosed in the present embodiment is a structure search device that searches for a stable structure of a compound in which a plurality of groups is linked, the device including a unit configured to arrange the plurality of groups at lattice points in a three-dimensional lattice space that is a set of a plurality of lattice points based on an objective function equation including a constraint term which is a term for making a coefficient value to a predetermined value, the constraint term expressing an inter-group distance with reference a shortest distance among distances between lattice points of a plurality of lattice points in the three-dimensional lattice space, the inter-group distance being a distance between a first group among the plurality of groups, the first group being arranged at a first lattice point among the plurality of lattice points, and a second group that is one of the plurality of groups, the second group being arranged at a second lattice point among the plurality of lattice points, and is linked to the first group, and creates a three-dimensional structure of the compound in the three-dimensional lattice space by the arrange.

The structure search device disclosed in the present embodiment includes a unit that creates the three-dimensional structure (three-dimensional structure creation unit) and further includes another unit (unit) as needed.

The structure search device includes, for example, a memory and a processor, and further includes other units as needed. As the processor, a processor coupled to a memory can be favorably used so that the process of creating a three-dimensional structure can be executed.

The processor can be, for example, a central processing unit (CPU), a graphics processing unit (GPU), or a combination thereof.

As described above, the structure search device disclosed in the present embodiment can be, for example, a device (computer) that executes the structure search program disclosed in the present embodiment. Therefore, a suitable mode in the structure search device disclosed in the present embodiment can be made similar to the suitable mode in the structure search program disclosed in the present embodiment.

(Computer-Readable Recording Medium)

A computer-readable recording medium disclosed in the present embodiment records the structure search program disclosed in the present embodiment.

The computer-readable recording medium disclosed in the present embodiment is not limited to any particular medium and can be appropriately selected according to the purpose. Examples of the computer-readable recording medium include a built-in hard disk, an external hard disk, a CD-ROM, a DVD-ROM, an MO disk, a USB memory, and the like.

Furthermore, the computer-readable recording medium disclosed in the present embodiment may be a plurality of recording media in which the structure search program disclosed in the present embodiment is divided and recorded for each of any pieces of processing.

Hereinafter, an example of the technology disclosed in the present embodiment will be described in more detail using configuration examples of the device, flowcharts, and the like.

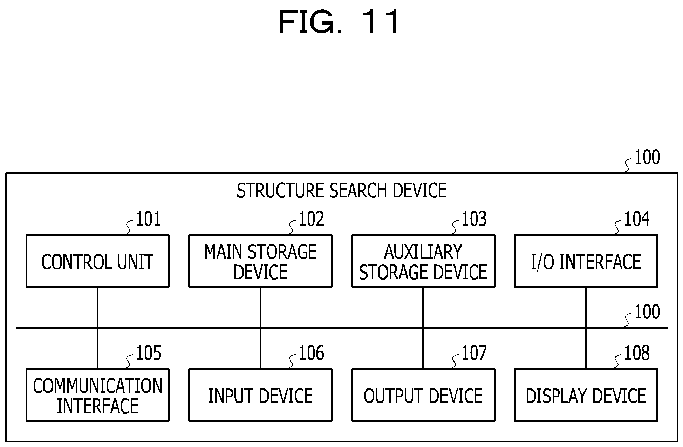

FIG. 11 illustrates a hardware configuration example of the structure search device disclosed in the present embodiment.

In a structure search device 100, for example, a control unit 101, a main storage device 102, an auxiliary storage device 103, an I/O interface 104, a communication interface 105, an input device 106, an output device 107, and a display device 108 are connected to one another via a system bus 109.

The control unit 101 performs arithmetic operations (for example, four arithmetic operations, comparison operations, and arithmetic operations for the annealing method), hardware and software operation control, and the like. The control unit 101 may be, for example, a central processing unit (CPU), a part of the annealing machine used for the annealing method, or a combination thereof.

The control unit 101 implements various functions, for example, by executing the program (for example, the structure search program disclosed in the present embodiment or the like) read in the main storage device 102 or the like.

Processing executed by the three-dimensional structure creation unit in the structure search device disclosed in the present embodiment can be executed by, for example, the control unit 101.

The main storage device 102 stores various programs and data or the like needed for executing various programs. As the main storage device 102, for example, a device having at least one of a read only memory (ROM) and a random access memory (RAM) can be used.

The ROM stores various programs, for example, a basic input/output system (BIOS) or the like. Furthermore, the ROM is not particularly limited and can be appropriately selected according to the purpose. For example, a mask ROM, a programmable ROM (PROM), or the like can be exemplified.

The RAM functions, for example, as a work range expanded when various programs stored in the ROM, the auxiliary storage device 103, or the like are executed by the control unit 101. The RAM is not particularly limited and can be appropriately selected according to the purpose. For example, a dynamic random access memory (DRAM), a static random access memory (SRAM), or the like can be exemplified.

The auxiliary storage device 103 is not particularly limited as long as the device can store various information and can be appropriately selected according to the purpose. For example, a solid state drive (SSD), a hard disk drive (HDD), or the like can be exemplified. Furthermore, the auxiliary storage device 103 may be a portable storage device such as a CD drive, a DVD drive, or a Blu-ray (registered trademark) disc (BD) drive.

Furthermore, the structure search program disclosed in the present embodiment is, for example, stored in the auxiliary storage device 103, loaded into the RAM (main memory) of the main storage device 102, and executed by the control unit 101.

The I/O interface 104 is an interface used to connect various external devices. The I/O interface 104 can input/output data to/from, for example, a compact disc ROM (CD-ROM), a digital versatile disk ROM (DVD-ROM), a magneto-optical disk (MO disk), a universal serial bus (USB) memory (USB flash drive), or the like.

The communication interface 105 is not particularly limited, and a known communication interface can be appropriately used. For example, a communication device using wireless or wired communication or the like can be exemplified.

The input device 106 is not particularly limited as long as the device can receive input of various requests and information with respect to the structure search device 100, a known device can be appropriately used. For example, a keyboard, a mouse, a touch panel, a microphone, or the like can be exemplified. Furthermore, in a case where the input device 106 is a touch panel (touch display), the input device 106 can also serve as the display device 108.

The output device 107 is not particularly limited, and a known device can be appropriately used. For example, a printer or the like can be exemplified.

The display device 108 is not particularly limited, and a known device can be appropriately used. For example, a liquid crystal display, an organic EL display, or the like can be exemplified.

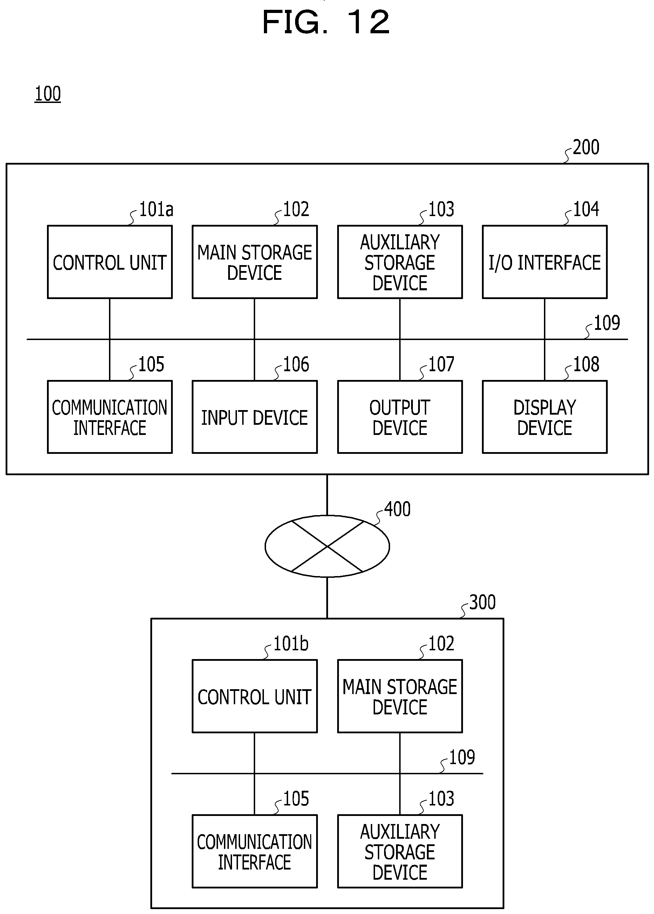

FIG. 12 illustrates another hardware configuration example of the structure search device disclosed in the present embodiment.

In the example illustrated in FIG. 12, the structure search device 100 is divided into a computer 200 that performs processing for, for example, defining the objective function equation and an annealing machine 300 that performs optimization (ground state search) in the Ising model equation. Furthermore, in the example illustrated in FIG. 12, the computer 200 and the annealing machine 300 in the structure search device 100 are connected via a network 400.

In the example illustrated in FIG. 12, for example, as a control unit 101a of the computer 200, a CPU or the like can be used, and as a control unit 101b of the annealing machine 300, a device specialized in the annealing method (annealing) can be used.

In the example illustrated in FIG. 12, for example, the computer 200 sets various settings for defining the objective function equation, defines the objective function equation, and converts the defined objective function equation into the Ising model equation. Then, information regarding values of a weight ($w_{ij}$) and a bias ($b_i$) in the Ising model equation is transmitted from the computer 200 to the annealing machine 300 via the network 400.

Next, the annealing machine 300 optimizes (minimize) the Ising model equation on the basis of the received information regarding the values of the weight ($w_{ij}$) and the bias ($b_i$) and obtains a minimum value of the Ising model equation and a state (state) of a bit that gives the minimum value. Then, the obtained minimum value of the Ising model equation and the obtained state (state) of the bit that gives the minimum value are transmitted from the annealing machine 300 to the computer 200 via the network 400.

Then, the computer 200 obtains the stable structure of the compound and the like on the basis of the state of the bit that gives the minimum value to the received Ising model equation.

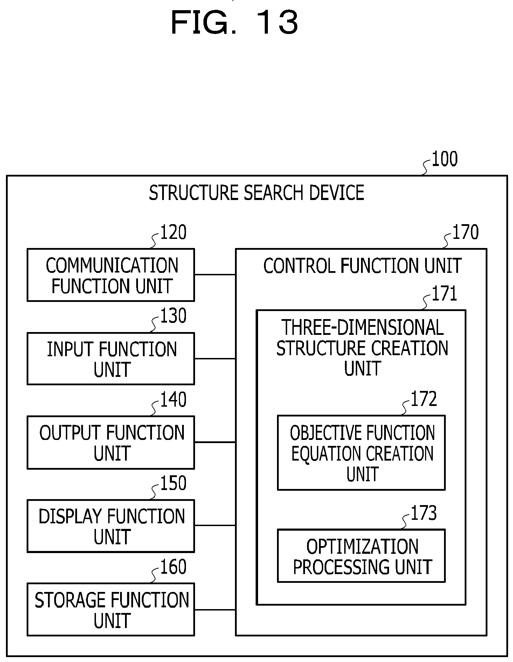

FIG. 13 illustrates an example of a functional configuration of the structure search device disclosed in the present embodiment.

As illustrated in FIG. 13, the structure search device 100 includes a communication function unit 120, an input function unit 130, an output function unit 140, a display function unit 150, a storage function unit 160, and a control function unit 170.

The communication function unit 120, for example, transmits and receives various data to and from an external device. The communication function unit 120 may receive structure data of the compound for which the stable structure is to be searched, data regarding a bias and a weight in an objective function equation converted into an Ising model equation from the external device, for example.

The input function unit 130 accepts, for example, various instructions for the structure search device 100. Furthermore, the input function unit 130 may receive an input of structure data of the compound for which the stable structure is to be searched, data regarding a bias and a weight in an objective function equation converted into an Ising model equation, or the like, for example.

The output function unit 140 prints and outputs, for example, the data of the searched stable structure of the compound or the like.

The display function unit 150 displays, for example, the data of the searched stable structure of the compound on a display or the like.

The storage function unit 160 stores, for example, various programs, structure data of the compound for which the stable structure is to be searched, the data of the searched stable structure of the compound, or the like.

The control function unit 170 has a three-dimensional structure creation unit 171.

The three-dimensional structure creation unit 171 arranges the plurality of groups at the lattice points in the three-dimensional lattice space that is a set of the plurality of lattice points, and creates the three-dimensional structure of the compound in the three-dimensional lattice space, for example.

Furthermore, three-dimensional structure creation unit 171 expresses the inter-group distance between the first group among the plurality of groups, the first group being arranged at the first lattice point among the plurality of lattice points, and the second group that is one of the plurality of groups, the second group being arranged at the second lattice point among the plurality of lattice points, and is linked to the first group, in the three-dimensional lattice space, by the coefficient value with reference to the shortest distance among the distances between lattice points of the plurality of lattice points. Then, the three-dimensional structure creation unit 171 arranges the plurality of groups at the lattice points on the basis of the objective function equation including the constraint term that causes the coefficient value to become a predetermined value, and creates the three-dimensional structure of the compound in the three-dimensional lattice space.

The three-dimensional structure creation unit 171 includes an objective function equation creation unit 172 and an optimization processing unit 173.

The objective function equation creation unit 172 creates, for example, the objective function equation to be used for creating the three-dimensional structure of the compound, and converts the created objective function equation into the Ising model equation. The optimization processing unit 173 calculates the minimum energy of the Ising model equation by executing, for example, the ground state search using the annealing method for the Ising model equation.

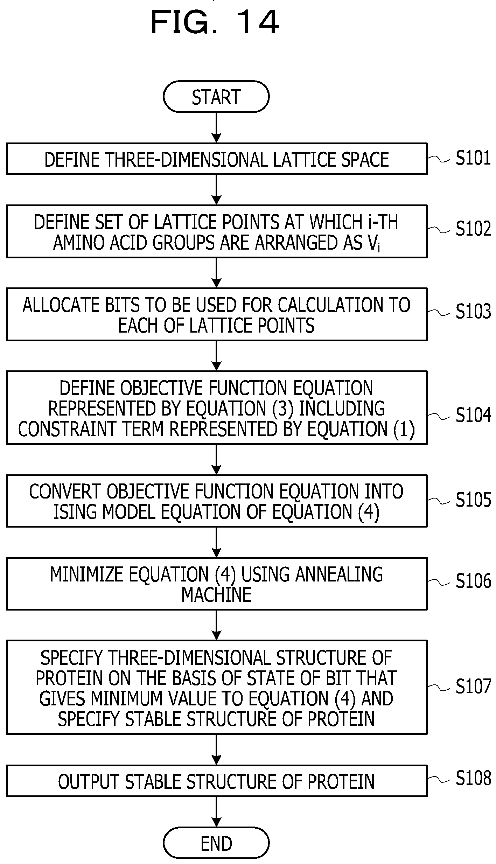

FIG. 14 illustrates an example of a flowchart when searching for a stable structure of a protein using an example of the technology disclosed in the present embodiment.

First, the three-dimensional structure creation unit 171 defines the three-dimensional lattice space (S101). More specifically, in S101, the three-dimensional structure creation unit 171 defines the three-dimensional lattice space that is a set of the lattice points at which the plurality of amino acid residues is arranged based on the number of amino acid residues in the protein for which the stable structure is to be searched.

Here, an example of the definition of the three-dimensional lattice space will be described. Note that the lattice space is a three-dimensional space, but hereinafter, a two-dimensional case will be described as an example for simplification.

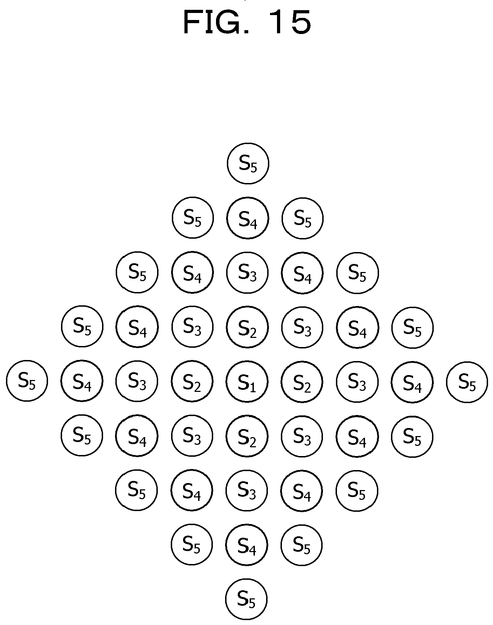

First, it is assumed that a set of lattices having a radius r in the diamond lattice space is Shell, and each lattice point is $S_r$. Then, each lattice point $S_r$ can be represented as illustrated in FIG. 15.

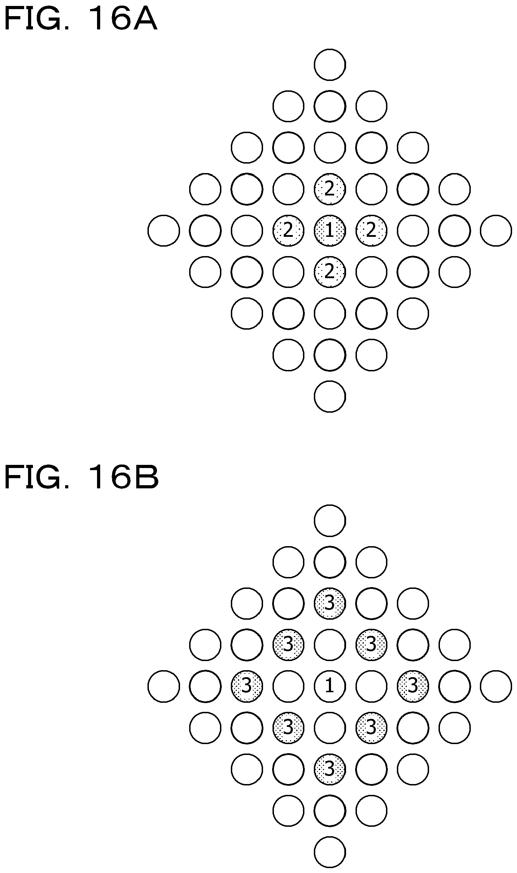

When each lattice point $S_r$ is defined as illustrated in FIG. 15, for example, a set of lattice points $V_1$ to $V_5$ at which the first to fifth amino acid residues are arranged is illustrated in FIGs. 16A to 16D.

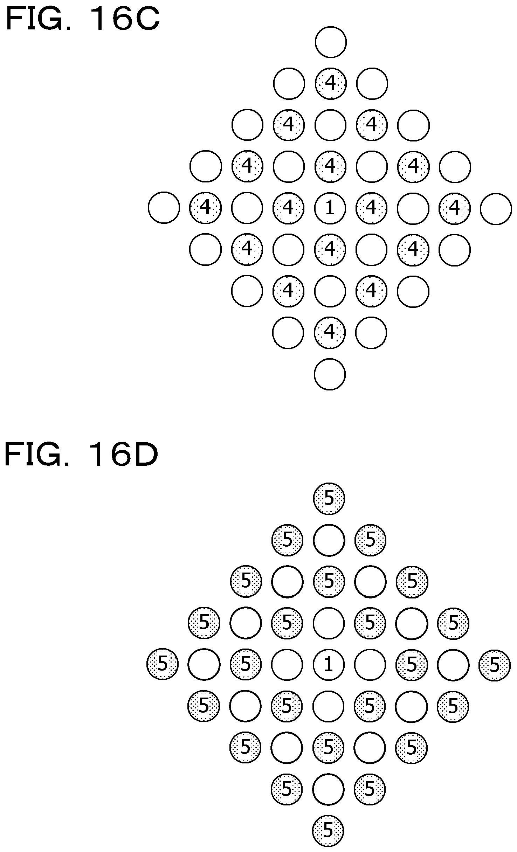

Here, in FIG. 16A, $V_1 = S_1$ and $V_2 = S_2$. Similarly, in FIG. 16B, $V_3 = S_3$. In FIG. 16C, $V_4 = S_2$ and $S_4$, and in FIG. 16D, $V_5 = S_3$ and $S_5$.

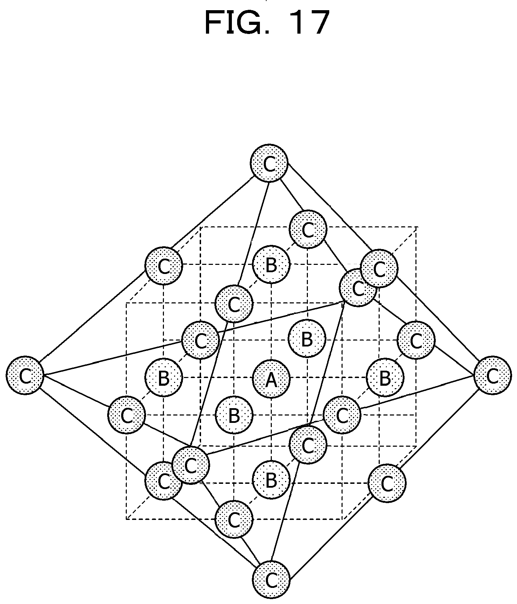

Note that $S_1$, $S_2$, and $S_3$ are three-dimensionally illustrated as in FIG. 17. In FIG. 17, A = $S_1$, B = $S_2$, and C = $S_3$.

Furthermore, a space $V_i$ needed for the i-th amino acid residue in the protein having n amino acid residues is represented by the following equation.

$$V_i = \bigcup_{r \in J} S_r$$

Here, i = {1, 2, 3,......, n}.

Then, in the case of odd-numbered (i = an odd number) amino acid residues, J = {1, 3,....., i}, and in the case of even-numbered (i = an even number) amino acid residues, J = {2, 4,....., i}.

Then, returning to FIG. 14, the three-dimensional structure creation unit 171 defines the set of lattice points at which the i-th amino acid residues are arranged as $V_i$ (S102). In S102, the space where the amino acid residues are placed is defined by defining the set of lattice points at which the i-th amino acid residues are arranged as $V_i$.

Next, the three-dimensional structure creation unit 171 allocates bits to be used for calculation to the lattice points (S103). In other words, in S103, the three-dimensional structure creation unit 171 allocates spatial information to bits $X_1$ to $X_n$.

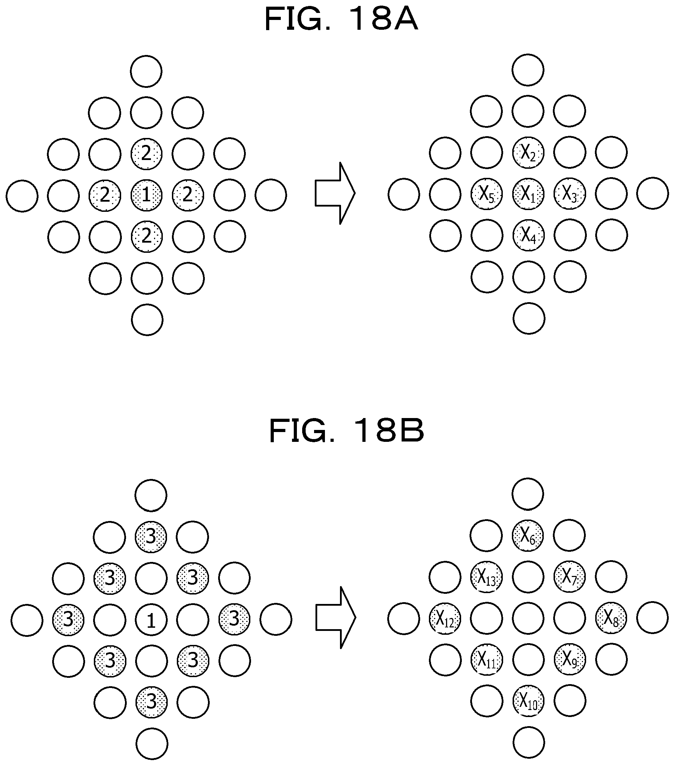

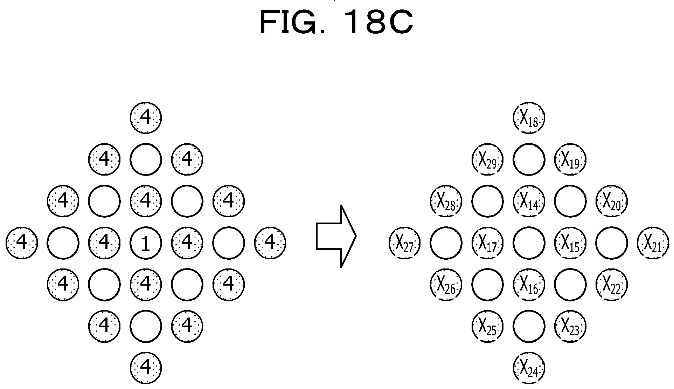

Specifically, as illustrated in FIGs. 18A to 18C, the bit representing the presence of the amino acid residue in a lattice point as "1" and the bit representing the absence of the amino acid residue in a lattice point as "0" are allocated to the space where the amino acid residues are arranged. Note that, in FIGs. 18A to 18C, for convenience of description, a plurality of $X_i$ is allocated to the amino acid residues 2 to 4, but in reality, one bit $X_i$ is allocated to one amino acid residue.

Next, returning to FIG. 14, the three-dimensional structure creation unit 171 defines the objective function equation represented by the following equation (3), including the constraint term represented by the following equation (1) (S104).

$$H_{conn} = \sum_n \left[ \sum_{i \in a(n), j \in a(n+1)} \{abs(d_{ij} - d_0) q_i q_j \} \right] \quad ... \text{ Equation (1)}$$

Note that, in the equation (1), $H_{conn}$ is the constraint term for causing the coefficient value to be a predetermined value, $a(n)$ is a set of bit numbers in the n-th group, $a(n + 1)$ is a set of bit numbers in the (n + 1)-th group, $d_{ij}$ is the inter-group distance between a group arranged at an i-th lattice point of the plurality of lattice points and a group arranged at a j-th lattice point of the plurality of lattice points, $d_0$ is the shortest distance, $abs(d_{ij} - d_0)$ is the coefficient value represented by an absolute value of a difference between $d_{ij}$ and $d_0$, $q_i$ is a binary variable of 0 or 1 that represents the presence or absence of the group arranged at the i-th lattice point, and $q_j$ is a binary variable of 0 or 1 that represents the presence or absence of the group arranged at the j-th lattice point.

$$H_{total} = \{ \lambda_{one} \times H_{one} + \lambda_{olap} \times H_{olap} + \lambda_{conn} \times (H_{conn} + C) \} + H_{pair} \quad ... \text{ Equation (3)}$$

Note that, in the equation (3), $H_{one}$ is the constraint term representing the constraint that the number of each of the plurality of groups is only one, $\lambda_{one}$ is the parameter for weighting the $H_{one}$, $H_{olap}$ is the constraint term representing the constraint that the plurality of groups does not overlap with one another, $\lambda_{olap}$ is the parameter for weighting the $H_{olap}$, $H_{conn}$ represents the constraint that the plurality of groups is connected to one another, and is the constraint term represented by the equation (1), C is the constant term regarding the constraint that the plurality of groups is connected to one another, $\lambda_{conn}$ is the parameter for weighting the $H_{conn}$ and the C, and $H_{pair}$ is the term representing the interaction between the plurality of groups.

Here, an example of each term in the above-described equation (3) will be described.

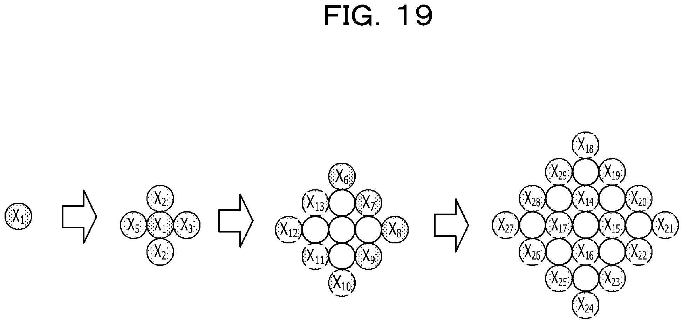

Note that, in FIGs. 19 to 21B to be described below, $X_1$ represents a position where the amino acid residue of the number 1 can be arranged. $X_2$ to $X_5$ represent positions where the amino acid residue of the number 2 can be arranged. $X_6$ to $X_{13}$ represent positions where the amino acid residue of the number 3 can be arranged. $X_{14}$ to $X_{29}$ represent positions where the amino acid residue of the number 4 can be arranged.

An example of the $H_{one}$ is described below.

$$H_{one} = \sum_{i=0}^{N-1} \sum_{x_a, x_b \in Q_i, a<b} x_a x_b$$

In the above-described $H_{one}$, $X_a$ and $X_b$ take 1 or 0. That is, in FIG. 19, since only one of $X_2$, $X_3$, $X_4$, and $X_5$ is 1, the $H_{one}$ is a function in which the energy increases when any two or more of the $X_2$, $X_3$, $X_4$, and $X_5$ is 1, and is a term of penalty that becomes 0 when only one of the $X_2$, $X_3$, $X_4$, and $X_5$ is 1.

An example of the $H_{olap}$ is described below.

$$H_{olap} = \sum_{v \in V} \sum_{x_a, x_b \in \theta(v), a<b} x_a x_b$$

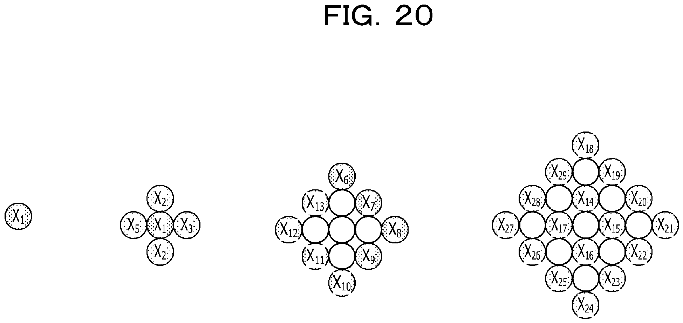

In the above-described $H_{olap}$, $X_a$ and $X_b$ take 1 or 0. That is, the $H_{olap}$ is a term in which a penalty occurs in the case where $X_{14}$ becomes 1 when $X_2$ is 1 in FIG. 20.

An example of $H_{pair}$ is described below.

$$H_{pair} = \frac{1}{2} \sum_{i=0}^{N-1} \sum_{x_a \in Q_i} \sum_{x_b \in \eta(x_a)} P_{\omega(x_a)\omega(x_b)} x_a x_b$$

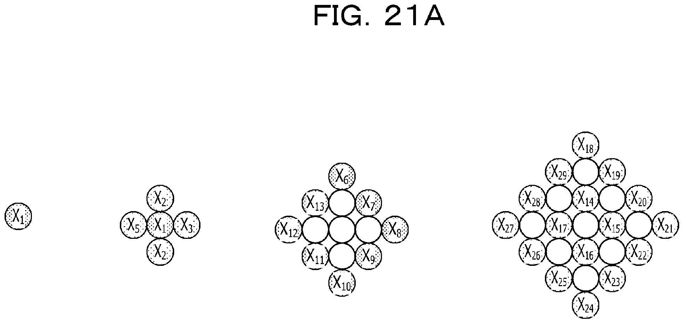

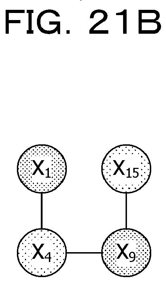

In the above-described $H_{pair}$, $X_a$ and $X_b$ take 1 or 0. That is, in FIGs. 21A and 21B, $H_{pair}$ is a function in which an interaction $P_{\omega(x1)\omega(x15)}$ works between the amino acid residue at $X_1$ and the amino acid residue at $X_{15}$ in the case where the $X_{15}$ becomes 1 when the $X_1$ is 1, and the energy decreases.

Then, returning to FIG. 14, the three-dimensional structure creation unit 171 converts the objective function equation into the Ising model equation of the equation (4) (S105). More specifically, in S105, the three-dimensional structure creation unit 171 converts the objective function equation into the Ising model equation expressed by the following equation (4) by extracting the parameters in the objective function equation and obtaining $b_i$ (bias) and $w_{ij}$ (weight) in the following equation (4).

$$E = - \sum_{i,j=0} w_{ij} x_i x_j - \sum_{i=0} b_i x_i \qquad \text{... Equation (4)}$$

Note that, in the above-described equation (4), E is the objective function equation converted into the Ising model equation.

$w_{ij}$ is a numerical value representing the interaction between the i-th bit and the j-th bit.
$b_i$ is a numerical value representing the bias for the i-th bit.
$x_i$ is a binary variable representing that the i-th bit is 0 or 1.
$w_j$ is a binary variable representing that the j-th bit is 0 or 1.

Next, the three-dimensional structure creation unit 171 minimizes the above-described equation (4), using the annealing machine (S106). In other words, in S106, the three-dimensional structure creation unit 171 specifies the state of the bit that gives the minimum value to the objective function equation by calculating the minimum value of the above-described equation (4) by executing the ground state search (optimization calculation) using the annealing method regarding the above-described equation (4).

Next, the three-dimensional structure creation unit 171 creates the three-dimensional structure of the protein on the basis of the state of the bit that gives the minimum value to the above-described equation (4) and specifies the stable structure of the protein (S107). More specifically, in S107, the three-dimensional structure creation unit 171 specifies the stable structure of the protein by arranging the amino acid residues in the three-dimensional lattice space and creating the three-dimensional structure of the protein on the basis of the state of the bit that gives the minimum value to the above-described equation (4).

Then, the three-dimensional structure creation unit 171 outputs the stable structure of the protein and terminates the processing (S108). Furthermore, the stable structure of the protein may be output as a three-dimensional structure diagram of the protein or may be output as coordinate information of each amino acid residue forming the protein.

Furthermore, in FIG. 14, the flow of the processing in an example of the technology disclosed in the present embodiment has been described according to a specific order. However, in the technology disclosed in the present embodiment, it is possible to appropriately switch an order of each steps in a technically possible range. Furthermore, in the technology disclosed in the present embodiment, a plurality of steps may be collectively performed in a technically possible range.

Examples of the annealing method and the annealing machine will be described below.

The annealing method is a method for probabilistically working out a solution using superposition of random number values and quantum bits. The following describes a problem of minimizing a value of an evaluation function to be optimized as an example. The value of the evaluation function is referred to as energy. Furthermore, in a case where the value of the evaluation function is maximized, the sign of the evaluation function only needs to be changed.

First, a process is started from an initial state in which one of discrete values is assigned to each variable. With respect to a current state (combination of variable values), a state close to the current state (for example, a state in which only one variable is changed) is selected, and a state transition therebetween is considered. An energy change with respect to the state transition is calculated. Depending on the value, it is probabilistically determined whether to adopt the state transition to change the state or not to adopt the state transition to keep the original state. In a case where an adoption probability when the energy goes down is selected to be larger than that when the energy goes up, it can be expected that a state change will occur in a direction that the energy goes down on average, and that a state transition will occur to a more appropriate state over time. Therefore, there is a possibility that an optimum solution or an approximate solution that gives energy close to the optimum value can be obtained finally.

If this is adopted when the energy goes down deterministically and is not adopted when the energy goes up, the energy change decreases monotonically in a broad sense with respect to time, but no further change occurs when reaching a local solution. As described above, since there are a very large number of local solutions in the discrete optimization problem, a state is almost certainly caught in a local solution that is not so close to an optimum value. Therefore, when the discrete optimization problem is solved, it is important to determine probabilistically whether to adopt the state.

In the annealing method, it has been proved that by determining an adoption (permissible) probability of a state transition as follows, a state reaches an optimum solution in the limit of infinite time (iteration count).

Hereinafter, a method for working out an optimum solution using the annealing method will be described step by step.

(1) For an energy change (energy reduction) value ($-\Delta E$) due to a state transition, a permissible probability p of the state transition is determined by any one of the following functions f().

$$p(\Delta E, T) = f(-\Delta E / T)$$ Equation (1-1)

$$f_{metro}(x) = \min(1, e^x)$$ (Metropolis method) Equation (1-2)

$$f_{Gibbs}(x) = \frac{1}{1 + e^{-x}}$$ (Gibbs method) Equation (1-3)

Here, the reference T is a parameter called a temperature value and can be changed as follows, for example.

(2) The temperature value T is logarithmically reduced with respect to an iteration count t as represented by the following equation.

$$T = \frac{T_0 \log(c)}{\log(t+c)} \quad \text{Equation (2)}$$

Here, $T_0$ is an initial temperature value, and is desirably a sufficiently large value depending on a problem.

In a case where the permissible probability p represented by the equation in (1) is used, if a steady state is reached after sufficient iterations, an occupation probability of each state follows a Boltzmann distribution for a thermal equilibrium state in thermodynamics.

Then, when the temperature is gradually lowered from a high temperature, an occupation probability of a low energy state increases. Therefore, it is considered that the low energy state is obtained when the temperature is sufficiently lowered. Since this state is very similar to a state change caused when a material is annealed, this method is referred to as the annealing method (or pseudo-annealing method). Note that probabilistic occurrence of a state transition that increases energy corresponds to thermal excitation in the physics.

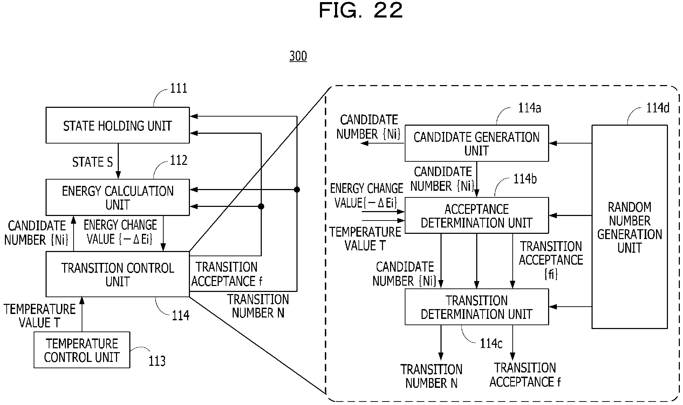

FIG. 22 illustrates an example of a functional configuration of an annealing machine that performs the annealing method. However, in the following description, a case of generating a plurality of state transition candidates is also described. However, a basic annealing method generates one transition candidate at a time.

The annealing machine 300 includes a state holding unit 111 that holds a current state S (a plurality of state variable values). Furthermore, the annealing machine 300 includes an energy calculation unit 112 that calculates an energy change value {-ΔEi} of each state transition when a state transition from the current state S occurs due to a change in any one of the plurality of state variable values. Moreover, the annealing machine 300 includes a temperature control unit 113 that controls the temperature value T and a transition control unit 114 that controls a state change. Note that the annealing machine 300 can be a part of the above-described structure search device 100.

The transition control unit 114 probabilistically determines whether or not to accept any one of a plurality of state transitions according to a relative relationship between the energy change value {-ΔEi} and thermal excitation energy, based on the temperature value T, the energy change value {-ΔEi}, and a random number value.

Here, the transition control unit 114 includes a candidate generation unit 114a that generates a state transition candidate, and an availability determination unit 114b for probabilistically determining whether or not to permit a state transition for each candidate based on the energy change value {-ΔEi} and the temperature value T. Moreover, the transition control unit 114 includes a transition determination unit 114c that determines a candidate to be adopted from the candidates that have been permitted, and a random number generation unit 114d that generates a random variable.

The operation of the annealing machine 300 in one iteration is as follows.

First, the candidate generation unit 114a generates one or more state transition candidates (candidate number {Ni}) from the current state S held in the state holding unit 111 to a next state. Next, the energy calculation unit 112 calculates the energy change value {-ΔEi} for each state transition listed as a candidate using the current state S and the state transition candidates. The availability determination unit 114b permits a state transition with a permissible probability of the above-described equation (1) according to the energy change value {-ΔEi} of each state transition using the temperature value T generated by the temperature control unit 113 and the random variable (random number value) generated by the random number generation unit 114d.

Then, the availability determination unit 114b outputs availability {fi} of each state transition. When there is a plurality of permitted state transitions, the transition determination unit 114c randomly selects one of the permitted state transitions using a random number value. Then, the transition determination unit 114c outputs a transition number N and transition availability f of the selected state transition. When there is a permitted state transition, a state variable value stored in the state holding unit 111 is updated according to the adopted state transition.

Starting from an initial state, the above-described iteration is repeated while the temperature value is lowered by the temperature control unit 113. When a completion determination condition such as reaching a certain iteration count or energy falling below a certain value is satisfied, the operation is completed. An answer output by the annealing machine 300 is a state when the operation is completed.

The annealing machine 300 illustrated in FIG. 22 may be implemented by using, for example, a semiconductor integrated circuit. For example, the transition control unit 114 may include a random number generation circuit that functions as the random number generation unit 114d, a comparison circuit that functions as at least a part of the availability determination unit 114b, a noise table to be described later, or the like.

Regarding the transition control unit 114 illustrated in FIG. 22, details of a mechanism that permits a state transition at a permissible probability represented in the equation in (1) will be further described.

A circuit that outputs 1 at the permissible probability p and outputs 0 at a permissible probability (1 - p) can be achieved by inputting the permissible probability p for input A and a uniform random number that takes a value of a section [0, 1) for input B in a comparator that has the two inputs A and B, and outputs 1 when A > B is satisfied and outputs 0 when A < B is satisfied. Therefore, if the value of the permissible probability p calculated based on the energy change value and the temperature value T using the equation in (1) is input to input A of this comparator, the above-described function can be achieved.

This means that, with a circuit that outputs 1 when $f(\Delta E/T)$ is larger than u, in which f is a function used in the equation in (1), and u is a uniform random number that takes a value of the section [0, 1), the above-described function can be achieved.

Furthermore, the same function as the above-described function can also be achieved by making the following modification.

Applying the same monotonically increasing function to two numbers does not change a magnitude relationship. Therefore, an output is not changed even if the same monotonically increasing function is applied to two inputs of the comparator. If an inverse function $f^{-1}$ of f is adopted as this monotonically increasing function, it can be seen that a circuit that outputs 1 when $-\Delta E/T$ is larger than $f^{-1}(u)$ can be adopted. Moreover, since the temperature value T is positive, it can be seen that a circuit that outputs 1 when $-\Delta E$ is larger than $Tf^{-1}(u)$ may be adopted.

The transition control unit 114 in FIG. 22 is a conversion table that realizes the inverse function $f^{-1}(u)$ and may include a noise table that outputs a value of a next function with respect to an input that is a discretized section [0, 1.

$$f_{metro}^{-1}(u) = \log(u) \qquad \text{Equation (3-1)}$$

$$f_{Gibbs}^{-1}(u) = \log\left(\frac{u}{1-u}\right) \qquad \text{Equation (3-2)}$$

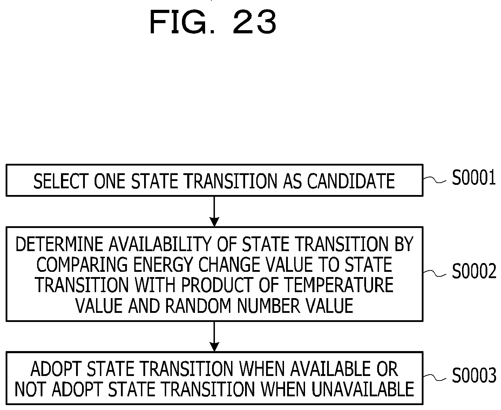

FIG. 23 is a diagram illustrating an exemplary operation flow of the transition control unit 114. The operation flow illustrated in FIG. 23 includes a step of selecting one state transition as a candidate (S0001), a step of determining availability of the state transition by comparing an energy change value for the state transition with a product of a temperature value and a random number value (S0002), and a step of adopting the state transition when the state transition is available, and not adopting the state transition when the state transition is not available (S0003).

[Embodiment]

Hereinafter, specific examples of the present embodiment and comparative examples with respect to the present embodiment will be described. Note that the present embodiment is not limited to the examples.

(Comparative Example 1)

First, as Comparative Example 1, the stable structure of Chignolin was searched applying the objective function equation (represented by the mathematical equation described with reference to FIG. 5 as $H_{conn}$ in the equation (3) to be described below) of the prior art in S104 using the flowchart illustrated in FIG. 14 using the structure search device as illustrated in FIG. 12. Furthermore, a digital annealer (registered trademark) was used as the annealing machine.

Note that the Chignolin used in Comparative Example 1 is a mutant of Chignolin represented by "YYDPETGTWY" when using one-letter notation of amino acid residues. Furthermore, details of Chignolin (PDB ID: 2RVD) used in Comparative Example 1 can be confirmed at "https://www.rcsb.org/structure/2RVD". In Comparative Example 1, the structure was created as a 1-bead model of Chignolin (one amino acid residue was coarse-grained into one particle) using a simple cubic lattice as the three-dimensional lattice space, and the stable structure was searched.

In Comparative Example 1, as illustrated below, the objective function equation of the following equation (3) including a constraint term dependent on the other constraint terms ($H_{one}$ and $H_{olap}$) based on the relationship that the constraint term ($H_{conn}$) that the amino acid residues are connected to one another is established among the individual linked amino acid residues was used.

$$H_{total} = \{\lambda_{one} \times H_{one} + \lambda_{olap} \times H_{olap} + \lambda_{conn} \times (H_{conn} + C)\} + H_{pair} \quad \text{... Equation (3)}$$

Note that, in the equation (3), $H_{total}$ is the objective function equation, $H_{one}$ is the constraint term representing the constraint that the number of each of the plurality of groups is only one, $\lambda_{one}$ is the parameter for weighting the $H_{one}$, $H_{olap}$ is the constraint term representing the constraint that the plurality of groups does not overlap with one another,

$\lambda_{olap}$ is the parameter for weighting the $H_{olap}$, $H_{conn}$ is the constraint term that the plurality of groups is connected to one another, C is the constant term regarding the constraint that the plurality of groups is connected to one another, $\lambda_{conn}$ is the parameter for weighting the $H_{conn}$ and the C, and $H_{pair}$ is the term representing the interaction between the plurality of groups.

In Comparative Example 1, the constraint term of the following equation in which both the $q_i$ and $q_j$ are "1" and $H_{conn}$ becomes a negative value in the case where the amino acid residue ($q_i$) numbered 3 and the amino acid residue ($q_j$) numbered 4 are arranged at positions adjacent to each other, as illustrated in FIG. 5, was used as the $H_{conn}$.

$$H_{conn} -= q_i q_j$$

In Comparative Example 1, the structure was searched for 216 (6 × 6 × 6) patterns assuming that each parameter takes a value of an integer multiple of 5 from 5 to 30 as patterns of the parameters of $\lambda_{one}$, $\lambda_{olap}$ ($\lambda_{overlap}$), and $\lambda_{conn}$ ($\lambda_{connect}$) in the objective function equation of the above-described equation (3).

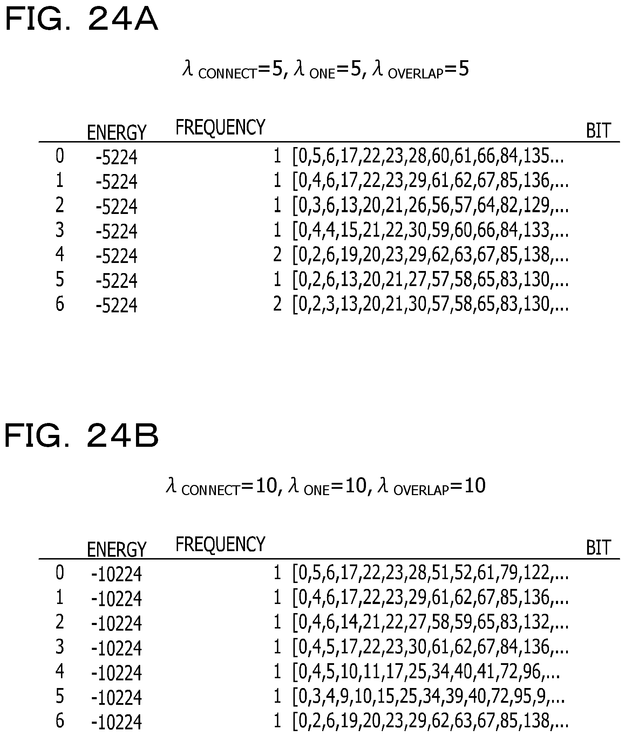

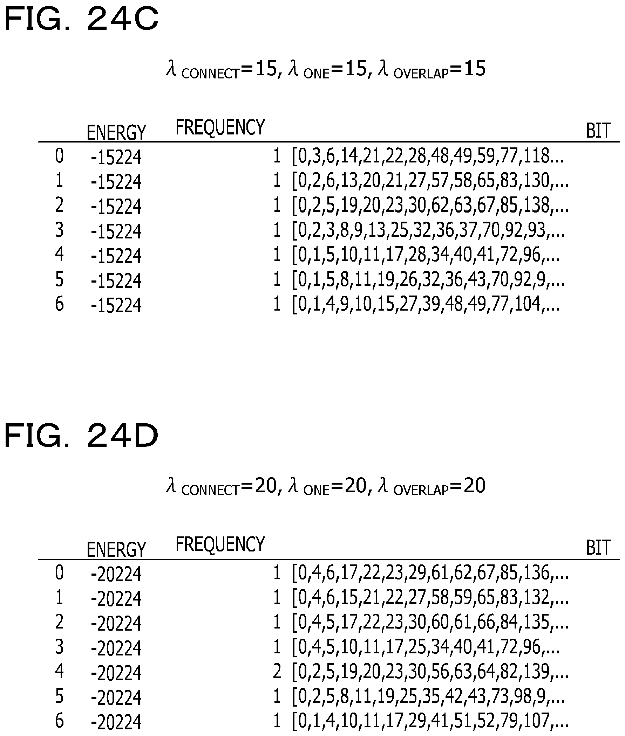

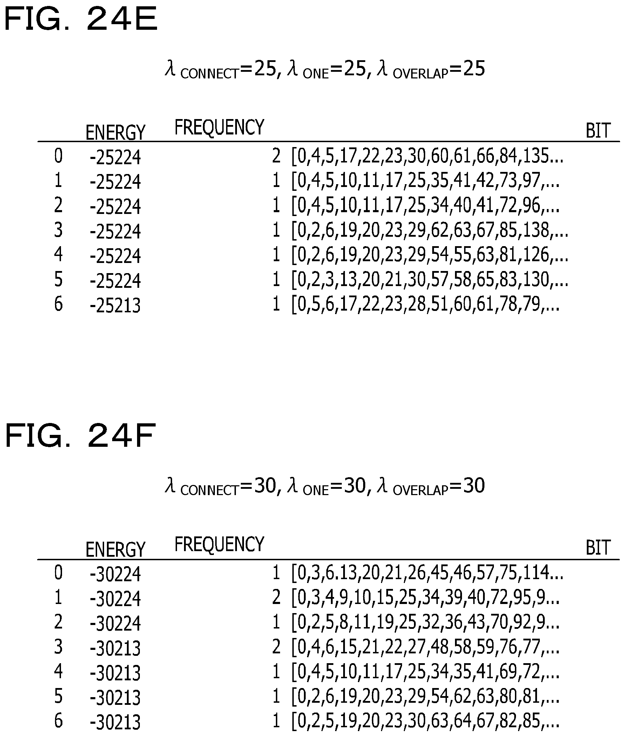

FIGs. 24A to 24F are diagrams illustrating an example of an energy value and bit numbers of "1" for seven types on a low energy side in a case of setting the parameters of $\lambda_{one}$, $\lambda_{olap}$ ($\lambda_{overlap}$), and $\lambda_{conn}$ ($\lambda_{connect}$) to the same value that is an integer multiple of 5 from 5 to 30 in Comparative Example 1. Furthermore, the low energy side means a side where the minimum value of the objective function equation is low, the energy value means the minimum value of the objective function equation, and the bit numbers of "1" means that the amino acid residue is arranged. Note that, in Comparative Example 1, the structure was searched under conditions of 20 parallels and the number of annealing iterations of 3 million. The vertical columns in FIGs. 24A to 24F mean results of parallel calculations different from one another.

Furthermore, the correct energy value (value of the objective function equation when Chignolin has the most stable structure) for Chignolin used in Comparative Example 1 was "-123" as a result of brute force calculation.

Note that, as processing of obtaining the correct energy value for Chignolin used in Comparative Example 1, first, processing of specifying the arrangement of the particle for all the lattice points having a possibility that the particle representing the next amino acid residue is arranged from the particle representing the amino acid residue existing at a certain lattice point was repeated until the arrangements of all the amino acid residues are completed. Then, a sum of interaction energies held by each other was calculated for the arrangements of the particles of all the cases, and the arrangement of the particle having the lowest energy was specified, so that the correct energy value (the energy value in the case of the most stable structure) was obtained.

As illustrated in FIGs. 24A to 24F, in the case of setting the parameters of $\lambda_{one}$, $\lambda_{olap}$, and $\lambda_{conn}$ to the same value that is an integer multiple of 5 from 5 to 30, there is no cases where the "Energy" is "-123". Furthermore, all the solutions (Energy) were values smaller than "-123", and were the solutions (structures) that do not satisfy the constraints of the objective function equation.

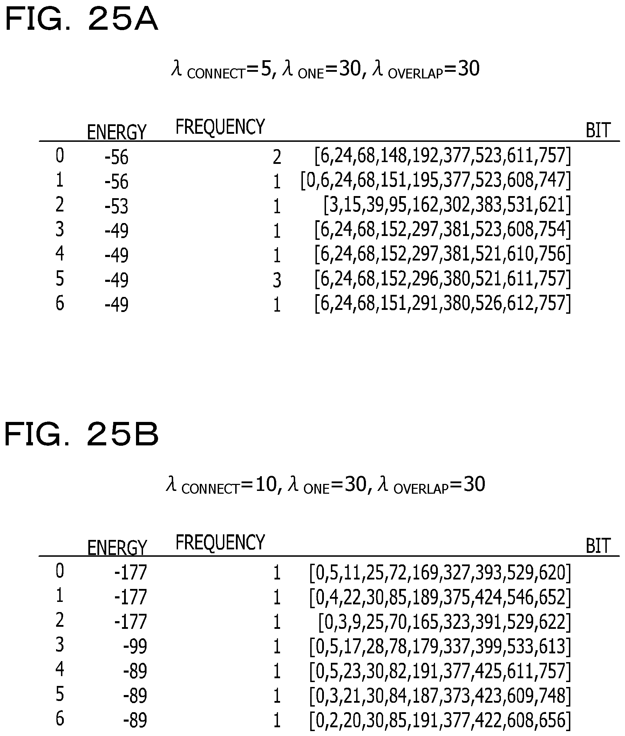

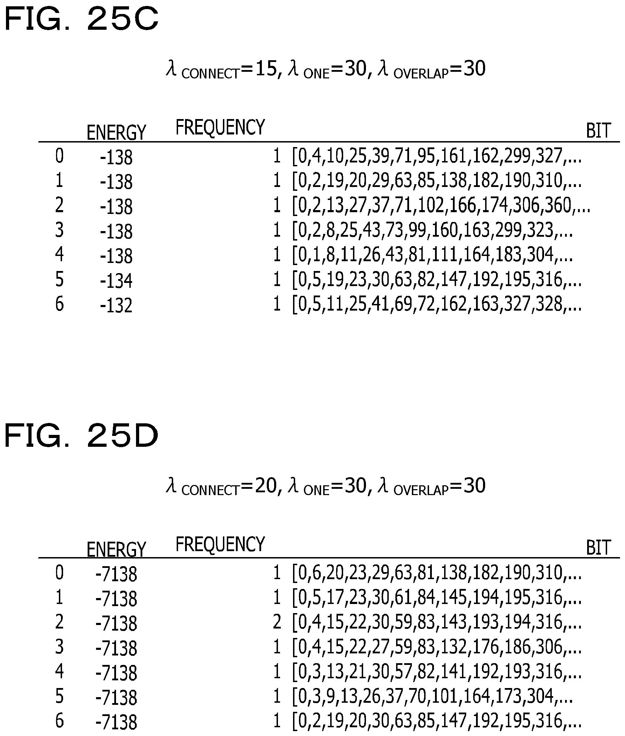

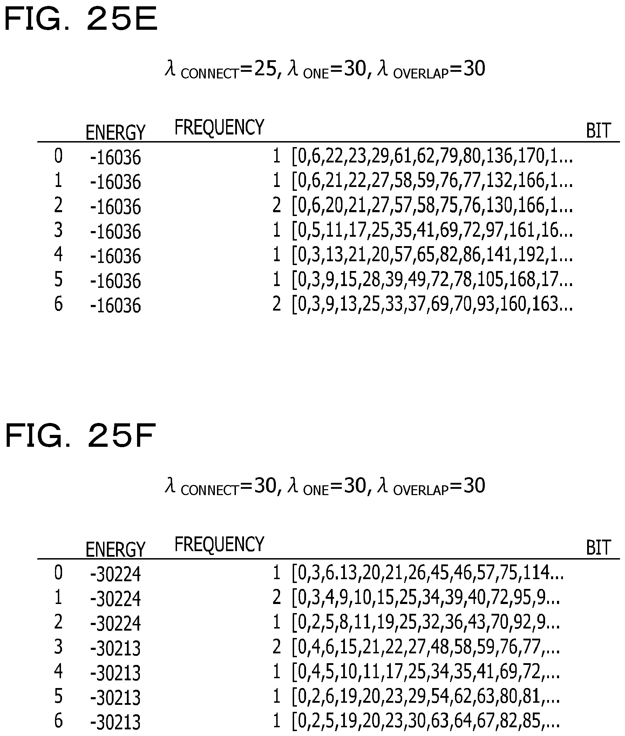

FIGs. 25A to 25F are diagrams illustrating an example of the energy value and the bit numbers of "1" for the seven types on the low energy side in a case of fixing $\lambda_{one}$ and $\lambda_{olap}$ to 30 and setting $\lambda_{conn}$ to an integer multiple of 5 from 5 to 30, among the parameters of $\lambda_{one}$, $\lambda_{olap}$, and $\lambda_{conn}$, in Comparative Example 1;

As illustrated in FIGs. 25A to 25F, in the case of fixing the parameters of $\lambda_{one}$ and $\lambda_{olap}$ to 30 and setting $\lambda_{conn}$ to an integer multiple of 5 from 5 to 30, there is no cases where the "Energy" is "-123". Furthermore, in the examples illustrated in FIGs. 25C to 25F, all the solutions (Energy) were values smaller than "-123", and were the solutions (structures) that do not satisfy the constraints of the objective function equation.

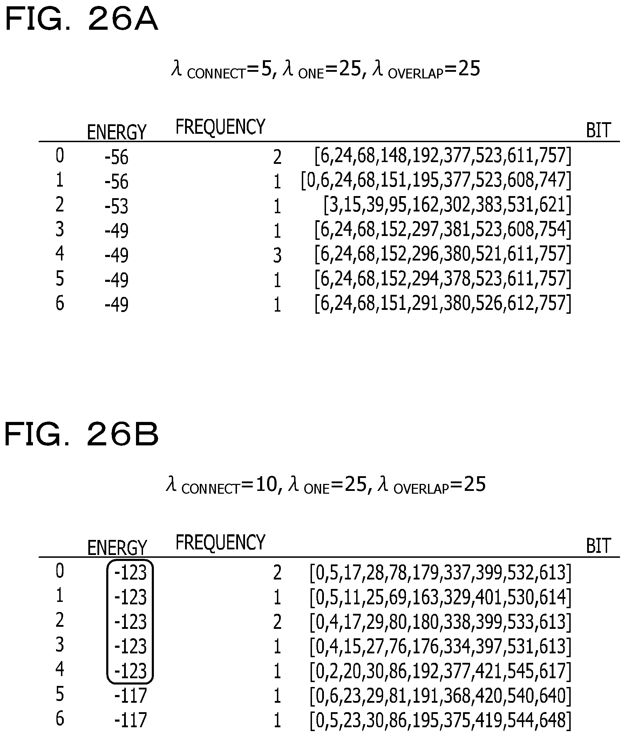

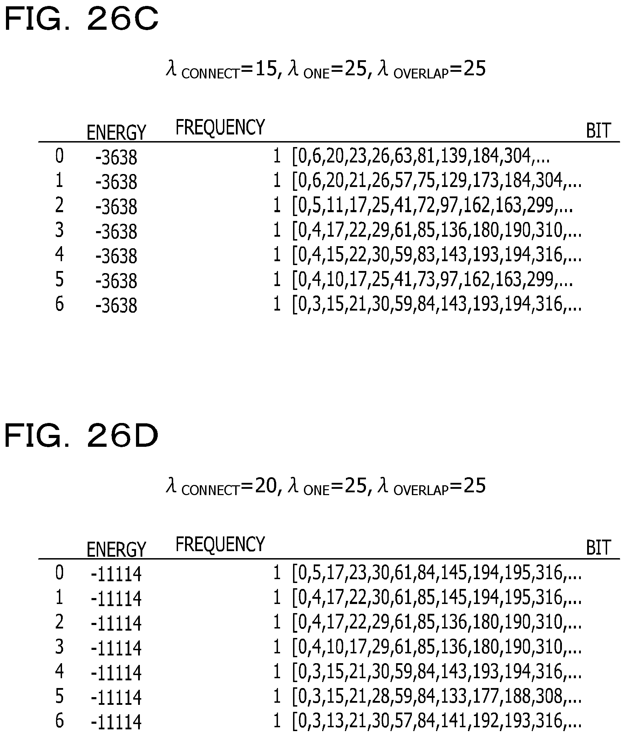

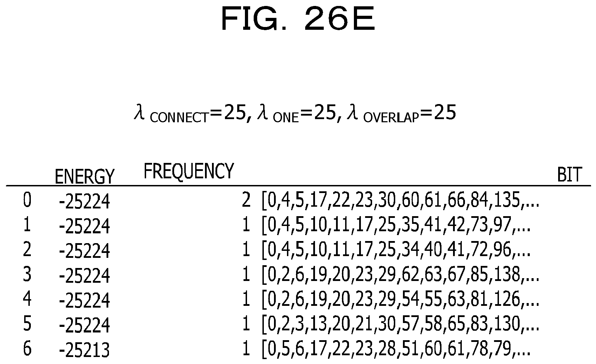

FIGs. 26A to 26E are diagrams illustrating an example of the energy value and the bit numbers of "1" for the seven types on the low energy side in a case of fixing $\lambda_{one}$ and $\lambda_{olap}$ to 25 and setting $\lambda_{conn}$ to an integer multiple of 5 from 5 to 25, among the parameters of $\lambda_{one}$, $\lambda_{olap}$, and $\lambda_{conn}$, in Comparative Example 1;

As illustrated in FIGs. 26A to 26E, in the case of fixing the parameters of $\lambda_{one}$ and $\lambda_{olap}$ to 25 and setting $\lambda_{conn}$ to 10 (FIG. 26B), the "Energy" was "-123", and the most stable structure for Chignolin was able to be searched. Furthermore, in the examples illustrated in FIGs. 26C to 26E, all the solutions (Energy) were values smaller than "-123", and were the solutions (structures) that do not satisfy the constraints of the objective function equation.

Furthermore, in patterns other than the patterns illustrated in FIG. 26B among the 216 patterns of the parameters of $\lambda_{one}$, $\lambda_{olap}$, and $\lambda_{conn}$, "Energy" was "-123" in only one pattern, and the most stable structure for Chignolin was able to be searched.

Thus, in Comparative Example 1, the most stable structure for Chignolin was able to be searched in only two patterns out of the 216 patterns of the parameters of $\lambda_{one}$, $\lambda_{olap}$, and $\lambda_{conn}$.

(Comparative Example 2)

In Comparative Example 2, the structure of Chignolin was created and the stable structure was searched, similarly to Comparative Example 1, except for using a constraint term (H) represented by the following equation as the $H_{conn}$. That is, in Comparative Example 2, the structure was searched with the independent relationships of $H_{one}$, $H_{olap}$, and $H_{conn}$.

$$H \mathrel{+}= (Q - q_0)(Q - 1)$$

$$Q = \Sigma_{i \in \eta(q_0)} q_i = q_1 + q_2 + q_3 + q_4$$

In the above-described equation, each of $q_0$, $q_1$, $q_2$, $q_3$, and $q_4$ takes "1" or "0". The positional relationship among the $q_0$, $q_1$, $q_2$, $q_3$, and $q_4$ is the positional relationship illustrated in FIG. 7.

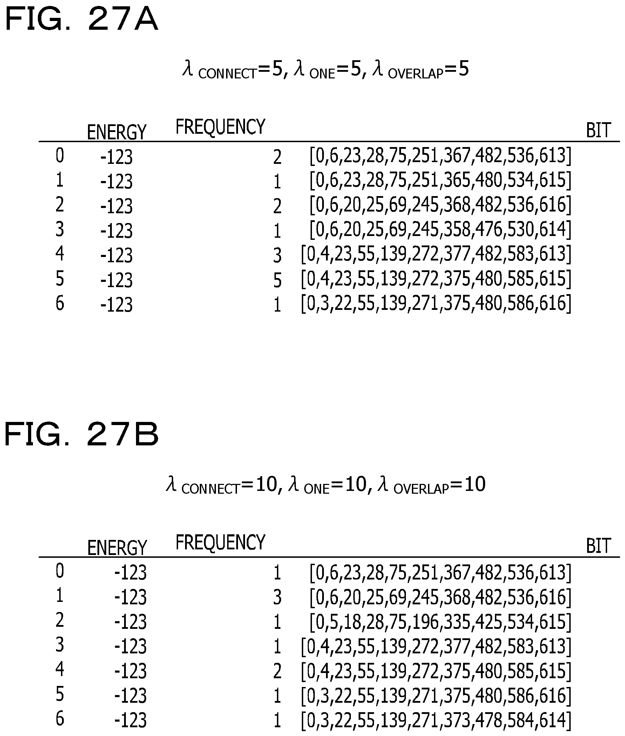

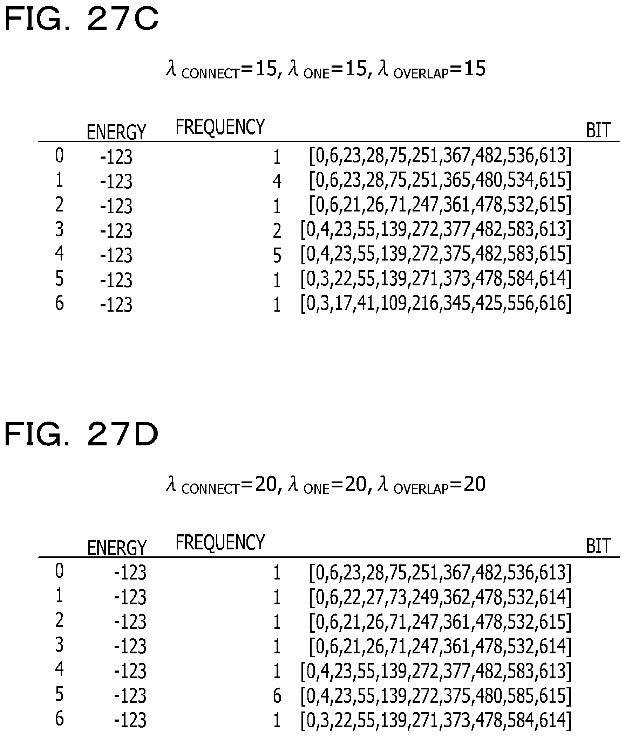

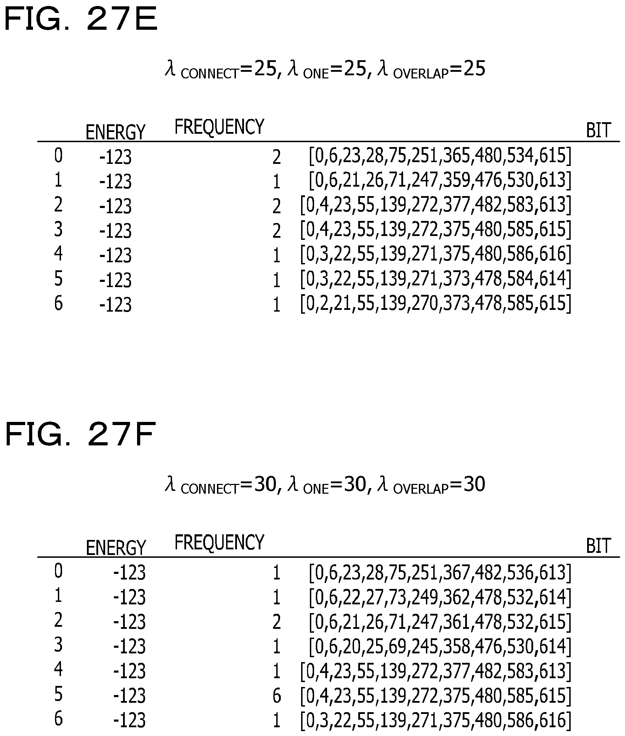

FIGs. 27A to 27F are diagrams illustrating an example of the energy value and the bit numbers of "1" for the seven types on the low energy side in a case of setting the parameters of $\lambda_{one}$, $\lambda_{olap}$, and $\lambda_{conn}$ to the same value that is an integer multiple of 5 from 5 to 30 in Comparative Example 2;

As illustrated in FIGs. 27A to 27F, in Comparative Example 2, the "Energy" was "-123" and the most stable structure for Chignolin was able to be searched, in all the patterns and all the parallel calculations.

Moreover, as Comparative Example 2, the structure was created and the stable structure was searched under conditions that the target for searching for the structure in Comparative Example 1 was changed to a cyclic peptide "PLP-2", the number of annealing iterations was set to $10^8$, and 100 parallels.

Furthermore, "PLP-2" used in Comparative Example 2 is a cyclic peptide represented by "DLFVPPID" when using one-letter notation of amino acid residues. Furthermore, details of "PLP-2" (PDB ID: 6AXI) used in Comparative Example 1 can be confirmed at "https://www.rcsb.org/structure/6AXI". In Comparative Example 2, the structure was created as a 2-bead model of "PLP-2" (one amino acid residue was coarse-grained into different particles of main chain and side chain) using a face-centered cubic lattice as the three-dimensional lattice space, and the stable structure was searched.

Note that, in Comparative Example 2, the parameters were set to $\lambda_{one}$ = 24, $\lambda_{olap}$ = 24, and $\lambda_{conn}$ = 15. The correct energy value (the value of the objective function equation in the case where PLP-2 has the most stable structure) for "PLP-2" used in Comparative Example 2 is "-436".

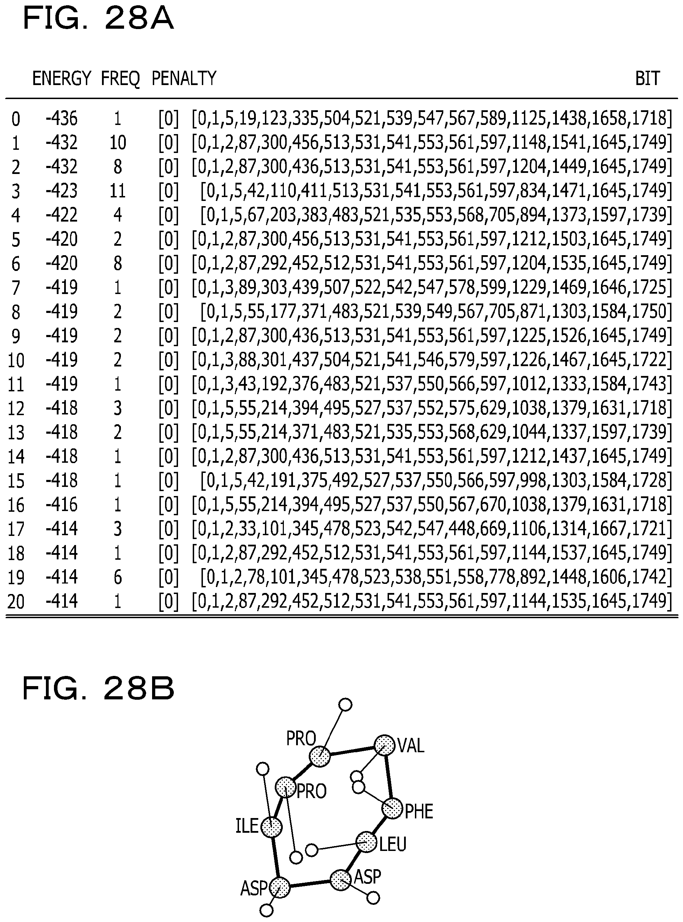

FIG. 28A is a diagram illustrating an example of the energy value and bit numbers of "1" for twenty types on the low energy side in Comparative Example 2.

As illustrated in FIG. 28A, in Comparative Example 2, the "Energy" was "-436" and the most stable structure for "PLP-2" was able to be searched in only one calculation out of 100 parallel calculations.

Furthermore, FIG. 28B illustrates the most stable structure of "PLP-2" obtained in Comparative Example 2;

(Embodiment 1)

The structure of "PLP-2" was created and the stable structure was searched similarly to Comparative Example 2 except for using a constraint term represented by the following equation (1) in which the coefficient value is expressed using the difference between the inter-group distance and the shortest distance as the $H_{conn}$ in Comparative Example 2 in which the structure of "PLP-2" was searched.

$$H_{conn} = \Sigma_n \left[ \Sigma_{i \in a(n), j \in a(n+1)} \{ abs(d_{ij} - d_0) q_i q_j \} \right] \quad \text{... Equation (1)}$$

Note that, in the equation (1), $H_{conn}$ is the constraint term for causing the coefficient value to be a predetermined value, a(n) is a set of bit numbers in the n-th group, a(n + 1) is a set of bit numbers in the (n + 1)-th group, $d_{ij}$ is the inter-group distance between the group arranged at the i-th lattice point and the group arranged at the j-th lattice point, $d_0$ is the shortest distance, $abs(d_{ij} - d_0)$ is the coefficient value represented by an absolute value of the difference between $d_{ij}$ and $d_0$, $q_i$ is a binary variable of 0 or 1 that represents the presence or absence of the group arranged at the i-th lattice point, and $q_j$ is a binary variable of 0 or 1 that represents the presence or absence of the group arranged at the j-th lattice point.

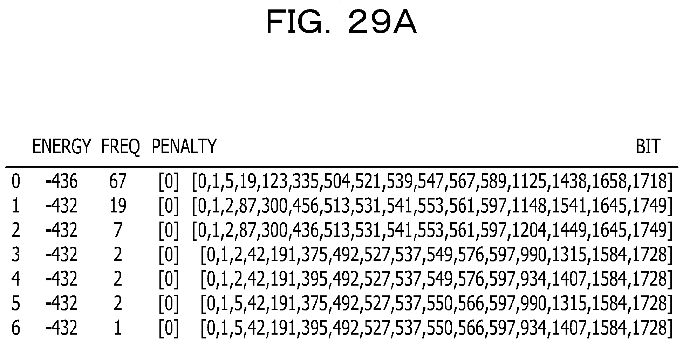

FIG. 29A is a diagram illustrating an example of the energy value and the bit numbers of "1" for seven types on the low energy side in Embodiment 1.

FIG. 29A illustrates the seven types on the low energy side, in Example 1, the "Energy" was "-436" and the most stable structure for "PLP-2" was able to be searched in 67 calculations out of 100 parallel calculations.

As described above, in Embodiment 1, the most stable structure was able to be searched in a larger number of parallel calculations than Comparative Example 2 in which the structure of "PLP-2" was searched, and the structure of "PLP-2" was able to be efficiently searched.

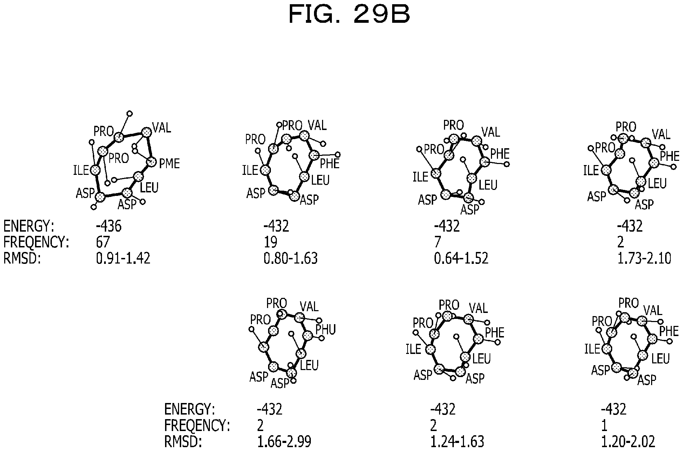

Furthermore, FIG. 29B is a diagram illustrating an example of a search result of the three-dimensional structure of "PLP-2" in Example 1. In FIG. 29B, "Energy" means the value of the objective function equation, and "Freq" means the number of parallel calculations in which the energy value was obtained out of 100 parallel calculations. Furthermore, in FIG. 29B, "Root Mean Square Deviation (RMSD)" means the magnitude of "misalignment" between the structure of PDB ID: 6AXI obtained by an experimental method (NMR) and the structure obtained by each calculation result. Furthermore, for each structure in the row of the "RMSD", the numerical value on the left side indicates the RMSD from the position of a $C\alpha$ carbon atom of each amino acid residue, and the numerical value on the right indicates the RMSD from the position of the side chain of each amino acid residue.

As illustrated in FIG. 29B, in Embodiment 1, the RMSD for the $C\alpha$ carbon atom between the structure obtained by 67 calculations out of the 100 parallel calculations and the structure of PDB ID: 6AXI obtained by NMR was 0.91. Moreover, as illustrated in FIG. 29B, in Embodiment 1, the RMSD for the $C\alpha$ carbon atom between the structure obtained by 19 calculations out of the 100 parallel calculations and the structure of PDB ID: 6AXI obtained by NMR was 0.80.

This result means that the stable structure searched in Embodiment 1 exhibits good match with the experimental structure identified by NMR.

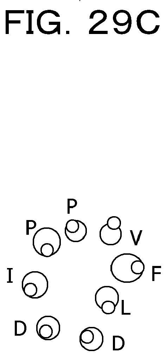

FIG. 29C is a diagram illustrating an example of the search result of the stable structure of "PLP-2" (the result of the energy value "-432") searched in Example 1 and the structure specified by NMR of the cyclic peptide superimposed each other. In FIG. 29C, a dark circle with a small diameter represents the position of the main chain ($C\alpha$ carbon atom) of each amino acid residue in the stable structure obtained in Embodiment 1, and a light circle with a large diameter represents the position of the $C\alpha$ carbon atom of each amino acid residue in PDB ID: 6AXI specified by NMR.

As illustrated in FIG. 29C, the stable structure of "PLP-2" obtained in Embodiment 1 exhibited good match with the structure of "PLP-2" specified by NMR. From the above, it was confirmed that the stable structure of "PLP-2" was able to be searched with high accuracy in Embodiment 1.

## Claims

1. A structure search program configured to search for a structure of a compound in which a plurality of groups is linked, the structure search program in which a computer performs a process of:

   determining an objective function including a constraint term which is a term for making a coefficient value to a predetermined value, the coefficient value expressing an inter-group distance with reference a shortest distance among distances between lattice points of a plurality of lattice points in a three-dimensional lattice space, the inter-group distance being a distance between a first group among the plurality of groups that is arranged at a first lattice point among the plurality of lattice points and a second group among the plurality of groups that is arranged at a second lattice point among the plurality of lattice points and is linked to the first group; and
   creating a three-dimensional structure of the compound in the three-dimensional lattice space by arranging the plurality of groups at lattice points in the three-dimensional lattice space that is a set of the plurality of lattice points based on the objective function .

2. The structure search program according to claim 1, wherein the constraint term is represented by an equation (1) below:

$$H_{conn} = \sum_n \left[ \sum_{i \in a(n), j \in a(n+1)} \{abs(d_{ij} - d_0) q_i q_j\} \right] \quad \text{... Equation (1)}$$

   where, in the equation (1),

   the $H_{conn}$ is a constraint term that causes the coefficient value to be a predetermined value,
   the $a(n)$ is a set of bit numbers in an n-th group,
   the $a(n + 1)$ is a set of bit numbers in an (n + 1)-th group,

the $d_{ij}$ is the inter-group distance between a group arranged at an i-th lattice point of the plurality of lattice points and a group arranged at a j-th lattice point of the plurality of lattice points,
the do is the shortest distance,
the $abs(d_{ij} - d_0)$ is the coefficient value represented by an absolute value of a difference between the $d_{ij}$ and the $d_0$,
the $q_i$ is a binary variable of 0 or 1 that represents presence or absence of the group arranged at the i-th lattice point, and
the $q_j$ is a binary variable of 0 or 1 that represents presence or absence of the group arranged at the j-th lattice point.

3. The structure search program according to claim 1, wherein the constraint term is represented by an equation (2) below:

$$H_{conn} = \sum_n \left[ \sum_{i \in a(n), j \in a(n+1)} \{abs\{(d_{ij}/d_0)-1\}q_i q_j\} \right] \quad \text{... Equation (2)}$$

where, in the equation (2),

the $H_{conn}$ is a constraint term that causes the coefficient value to be a predetermined value,
the $a(n)$ is a set of bit numbers in an n-th group of the plurality of groups,
the $a(n + 1)$ is a set of bit numbers in an (n + 1)-th group of the plurality of groups,
the $d_{ij}$ is the inter-group distance between a group arranged at the i-th lattice point and a group arranged at the j-th lattice point,
the do is the shortest distance,
the $abs\{(d_{ij}/d_0) - 1\}$ is the coefficient value represented by an absolute value of a number obtained by subtracting 1 from a ratio of the $d_{ij}$ and the $d_0$,
the $q_i$ is a binary variable of 0 or 1 that represents presence or absence of the group arranged at the i-th lattice point, and
the $q_j$ is a binary variable of 0 or 1 that represents presence or absence of the group arranged at the j-th lattice point.

4. The structure search program according to claim 2 or 3, wherein the creating includes creating the three-dimensional structure is performed by optimization processing based on the objective function which is represented by an equation (3) below:

$$H_{total} = \{\lambda_{one} \times H_{one} + \lambda_{olap} \times H_{olap} + \lambda_{conn} \times (H_{conn} + C)\} + H_{pair} \quad \text{... Equation (3)}$$

where, in the equation (3),

the $H_{total}$ is the objective function ,
the $H_{one}$ is a constraint term representing a constraint that the number of each of the plurality of groups is only one,
the $\lambda_{one}$ is a parameter to weight the $H_{one}$,
the $H_{olap}$ is a constraint term representing a constraint that the plurality of groups does not overlap with one another,
the $\lambda_{olap}$ is a parameter to weight the $H_{olap}$,
the $H_{conn}$ is a constraint representing that the plurality of groups is connected to one another, and is a constraint term represented by the equation (1) or the equation (2),
the C is a constant term regarding the constraint that the plurality of groups is connected to one another,
the $A_{conn}$ is a parameter to weight the $H_{conn}$ and the C, and
the $H_{pair}$ is a term representing an interaction between the plurality of groups.

5. The structure search program according to any one of claims 1 to 4, wherein the creating includes creating the three-dimensional structure is performed by optimization processing based on the objective function converted into an Ising model equation represented by an equation (4) below:

$$E = -\sum_{i,j=0} w_{ij}\, x_i x_j - \sum_{i=0} b_i x_i \qquad \text{... Equation (4)}$$

where, in the equation (4),

the E is the objective function converted into the Ising model equation,
the $w_{ij}$ is a numerical value that represents an interaction between an i-th bit and a j-th bit,
the $b_i$ is a numerical value that represents a bias with respect to the i-th bit,
the $x_i$ is a binary variable that represents that the i-th bit is 0 or 1, and
the $x_j$ is a binary variable that represents that the j-th bit is 0 or 1.

6. The structure search program according to claim 5, wherein the creating includes crating the three-dimensional structure is performed by specifying minimum energy of the Ising model equation by executing a ground state search using an annealing method, for the Ising model equation.

7. The structure search program according to any one of claims 1 to 6, wherein the compound is a protein or a peptide, and the plurality of groups is amino acid residues.

8. A structure search device that search for a structure of a compound in which a plurality of groups is linked, the structure search device comprising:
a creating unit configured to:

determine an objective function including a constraint term which is a term for making a coefficient value to a predetermined value, the coefficient value expressing an inter-group distance with reference a shortest distance among distances between lattice points of a plurality of lattice points in a three-dimensional lattice space, the inter-group distance being a distance between a first group among the plurality of groups that is arranged at a first lattice point among the plurality of lattice points and a second group among the plurality of groups that is arranged at a second lattice point among the plurality of lattice points and is linked to the first group; and
create a three-dimensional structure of the compound in the three-dimensional lattice space by arranging the plurality of groups at lattice points in the three-dimensional lattice space that is a set of the plurality of lattice points based on the objective function .

9. The structure search device according to claim 8, wherein the constraint term is represented by an equation (1) below:

$$H_{conn} = \sum_n \left[ \sum_{i \in a(n), j \in a(n+1)} \{abs(d_{ij} - d_0) q_i q_j\} \right] \qquad \text{... Equation (1)}$$

where, in the equation (1),

the $H_{conn}$ is a constraint term that causes the coefficient value to be a predetermined value,
the a(n) is a set of bit numbers in an n-th group,
the a(n + 1) is a set of bit numbers in an (n + 1)-th group,
the $d_{ij}$ is the inter-group distance between a group arranged at an i-th lattice point of the plurality of lattice points and a group arranged at a j-th lattice point of the plurality of lattice points,
the do is the shortest distance,
the $abs(d_{ij} - d_0)$ is the coefficient value represented by an absolute value of a difference between the $d_{ij}$ and the $d_0$,
the qi is a binary variable of 0 or 1 that represents presence or absence of the group arranged at the i-th lattice point, and
the $q_j$ is a binary variable of 0 or 1 that represents presence or absence of the group arranged at the j-th lattice point.

10. The structure search device according to claim 8, wherein the constraint term is represented by an equation (2) below:

$$H_{conn} = \sum_{n} \left[ \sum_{i \in a(n), j \in a(n+1)} \{abs\{(d_{ij}/d_0)-1\}q_i q_j\} \right] \quad \text{... Equation (2)}$$

where, in the equation (2),

the $H_{conn}$ is a constraint term that causes the coefficient value to be a predetermined value,
the $a(n)$ is a set of bit numbers in an n-th group of the plurality of groups,
the $a(n + 1)$ is a set of bit numbers in an (n + 1)-th group of the plurality of groups,
the $d_{ij}$ is the inter-group distance between a group arranged at the i-th lattice point and a group arranged at the j-th lattice point,
the $d_0$ is the shortest distance,
the $abs\{(d_{ij}/d_0) - 1\}$ is the coefficient value represented by an absolute value of a number obtained by subtracting 1 from a ratio of the $d_{ij}$ and the $d_0$,
the $q_i$ is a binary variable of 0 or 1 that represents presence or absence of the group arranged at the i-th lattice point, and
the $q_j$ is a binary variable of 0 or 1 that represents presence or absence of the group arranged at the j-th lattice point.

11. The structure search device according to claim 9 or 10, wherein the creating unit creates the three-dimensional structure is performed by optimization processing based on the objective function which is represented by an equation (3) below:

$$H_{total} = \{\lambda_{one} \times H_{one} + \lambda_{olap} \times H_{olap} + \lambda_{conn} \times (H_{conn} + C)\} + H_{pair} \quad \text{... Equation (3)}$$

where, in the equation (3),

the $H_{total}$ is the objective function ,
the $H_{one}$ is a constraint term representing a constraint that the number of each of the plurality of groups is only one,
the $\lambda_{one}$ is a parameter to weight the $H_{one}$,
the $H_{olap}$ is a constraint term representing a constraint that the plurality of groups does not overlap with one another,
the $\lambda_{olap}$ is a parameter to weight the $H_{olap}$,
the $H_{conn}$ is a constraint representing that the plurality of groups is connected to one another, and is a constraint term represented by the equation (1) or the equation (2),
the C is a constant term regarding the constraint that the plurality of groups is connected to one another,
the $\lambda_{conn}$ is a parameter to weight the $H_{conn}$ and the C, and
the $H_{pair}$ is a term representing an interaction between the plurality of groups.

12. The structure search device according to any one of claims 8 to 11, wherein the creating unit creates the three-dimensional structure is performed by optimization processing based on the objective function converted into an Ising model equation represented by an equation (4) below:

$$E = -\sum_{i,j=0} w_{ij} x_i x_j - \sum_{i=0} b_i x_i \quad \text{... Equation (4)}$$

where, in the equation (4),

the E is the objective function converted into the Ising model equation,
the $w_{ij}$ is a numerical value that represents an interaction between an i-th bit and a j-th bit,
the $b_i$ is a numerical value that represents a bias with respect to the i-th bit,
the $x_i$ is a binary variable that represents that the i-th bit is 0 or 1, and
the $x_j$ is a binary variable that represents that the j-th bit is 0 or 1.

**13.** The structure search device according to claim 5, wherein the creating unit crates the three-dimensional structure is performed by specifying minimum energy of the Ising model equation by executing a ground state search using an annealing method, for the Ising model equation.

**14.** The structure search device according to any one of claims 8 to 13, wherein the compound is a protein or a peptide, and the plurality of groups is amino acid residues.

**15.** A structure search method being performed by the structure search device that search for a structure of a compound in which a plurality of groups is linked, the structure search method comprising:

determining an objective function including a constraint term which is a term for making a coefficient value to a predetermined value, the coefficient value expressing an inter-group distance with reference a shortest distance among distances between lattice points of a plurality of lattice points in a three-dimensional lattice space, the inter-group distance being a distance between a first group among the plurality of groups that is arranged at a first lattice point among the plurality of lattice points and a second group among the plurality of groups that is arranged at a second lattice point among the plurality of lattice points and is linked to the first group; and
creating a three-dimensional structure of the compound in the three-dimensional lattice space by arranging the plurality of groups at lattice points in the three-dimensional lattice space that is a set of the plurality of lattice points based on the objective function .

FIG. 1A

FIG. 1B

FIG. 1C

# FIG. 2A

# FIG. 2B

# FIG. 2C

FIG. 2D

FIG. 2E

# FIG. 3

# FIG. 4

# FIG. 5

# FIG. 6

# FIG. 7

# FIG. 8

FUNCTION VALUE

LOCAL SOLUTION

BIT VARIABLE SPACE

# FIG. 9

LOCAL SOLUTION

FUNCTION VALUE

BIT VARIABLE SPACE

# FIG. 10

# FIG. 11

STRUCTURE SEARCH DEVICE — 100

| 101 CONTROL UNIT | 102 MAIN STORAGE DEVICE | 103 AUXILIARY STORAGE DEVICE | 104 I/O INTERFACE |

100

| 105 COMMUNICATION INTERFACE | 106 INPUT DEVICE | 107 OUTPUT DEVICE | 108 DISPLAY DEVICE |

# FIG. 12

100

# FIG. 13

~100

STRUCTURE SEARCH DEVICE

~120
COMMUNICATION FUNCTION UNIT

~130
INPUT FUNCTION UNIT

~140
OUTPUT FUNCTION UNIT

~150
DISPLAY FUNCTION UNIT

~160
STORAGE FUNCTION UNIT

~170
CONTROL FUNCTION UNIT

~171
THREE-DIMENSIONAL STRUCTURE CREATION UNIT

~172
OBJECTIVE FUNCTION EQUATION CREATION UNIT

~173
OPTIMIZATION PROCESSING UNIT

# FIG. 14

START

DEFINE THREE-DIMENSIONAL LATTICE SPACE — S101

DEFINE SET OF LATTICE POINTS AT WHICH i-TH AMINO ACID GROUPS ARE ARRANGED AS $V_i$ — S102

ALLOCATE BITS TO BE USED FOR CALCULATION TO EACH OF LATTICE POINTS — S103

DEFINE OBJECTIVE FUNCTION EQUATION REPRESENTED BY EQUATION (3) INCLUDING CONSTRAINT TERM REPRESENTED BY EQUATION (1) — S104

CONVERT OBJECTIVE FUNCTION EQUATION INTO ISING MODEL EQUATION OF EQUATION (4) — S105

MINIMIZE EQUATION (4) USING ANNEALING MACHINE — S106

SPECIFY THREE-DIMENSIONAL STRUCTURE OF PROTEIN ON THE BASIS OF STATE OF BIT THAT GIVES MINIMUM VALUE TO EQUATION (4) AND SPECIFY STABLE STRUCTURE OF PROTEIN — S107

OUTPUT STABLE STRUCTURE OF PROTEIN — S108

END

# FIG. 15

# FIG. 16A

# FIG. 16B

## FIG. 16C

## FIG. 16D

# FIG. 17

# FIG. 18A

# FIG. 18B

# FIG. 18C

# FIG. 19

# FIG. 20

# FIG. 21A

# FIG. 21B

# FIG. 22

300

STATE HOLDING UNIT 111

STATE S

ENERGY CALCULATION UNIT 112

CANDIDATE NUMBER {Ni}    ENERGY CHANGE VALUE {−ΔEi}

TRANSITION CONTROL UNIT

TRANSITION ACCEPTANCE f

TRANSITION NUMBER N

TEMPERATURE VALUE T    114

TEMPERATURE CONTROL UNIT 113

CANDIDATE NUMBER {Ni}    CANDIDATE GENERATION UNIT 114a

CANDIDATE NUMBER {Ni}

ENERGY CHANGE VALUE{−ΔEi}    ACCEPTANCE DETERMINATION UNIT 114b

TEMPERATURE VALUE T

RANDOM NUMBER GENERATION UNIT 114d

CANDIDATE NUMBER {Ni}    TRANSITION ACCEPTANCE {fi}

TRANSITION DETERMINATION UNIT

TRANSITION NUMBER N    TRANSITION ACCEPTANCE f    114c

# FIG. 23

| SELECT ONE STATE TRANSITION AS CANDIDATE | S0001 |

| DETERMINE AVAILABILITY OF STATE TRANSITION BY COMPARING ENERGY CHANGE VALUE TO STATE TRANSITION WITH PRODUCT OF TEMPERATURE VALUE AND RANDOM NUMBER VALUE | S0002 |

| ADOPT STATE TRANSITION WHEN AVAILABLE OR NOT ADOPT STATE TRANSITION WHEN UNAVAILABLE | S0003 |

# FIG. 24A

$\lambda_{CONNECT}=5, \lambda_{ONE}=5, \lambda_{OVERLAP}=5$

| | ENERGY | FREQUENCY | | BIT |
|---|---|---|---|---|
| 0 | -5224 | 1 | [0,5,6,17,22,23,28,60,61,66,84,135... | |
| 1 | -5224 | 1 | [0,4,6,17,22,23,29,61,62,67,85,136,... | |
| 2 | -5224 | 1 | [0,3,6,13,20,21,26,56,57,64,82,129,... | |
| 3 | -5224 | 1 | [0,4,4,15,21,22,30,59,60,66,84,133,... | |
| 4 | -5224 | 2 | [0,2,6,19,20,23,29,62,63,67,85,138,... | |
| 5 | -5224 | 1 | [0,2,6,13,20,21,27,57,58,65,83,130,... | |
| 6 | -5224 | 2 | [0,2,3,13,20,21,30,57,58,65,83,130,... | |

# FIG. 24B

$\lambda_{CONNECT}=10, \lambda_{ONE}=10, \lambda_{OVERLAP}=10$

| | ENERGY | FREQUENCY | | BIT |
|---|---|---|---|---|
| 0 | -10224 | 1 | [0,5,6,17,22,23,28,51,52,61,79,122,... | |
| 1 | -10224 | 1 | [0,4,6,17,22,23,29,61,62,67,85,136,... | |
| 2 | -10224 | 1 | [0,4,6,14,21,22,27,58,59,65,83,132,... | |
| 3 | -10224 | 1 | [0,4,5,17,22,23,30,61,62,67,84,136,... | |
| 4 | -10224 | 1 | [0,4,5,10,11,17,25,34,40,41,72,96,... | |
| 5 | -10224 | 1 | [0,3,4,9,10,15,25,34,39,40,72,95,9,... | |
| 6 | -10224 | 1 | [0,2,6,19,20,23,29,62,63,67,85,138,... | |

# FIG. 24C

$\lambda_{\text{CONNECT}}=15, \lambda_{\text{ONE}}=15, \lambda_{\text{OVERLAP}}=15$

| | ENERGY | FREQUENCY | | BIT |
|---|---|---|---|---|
| 0 | -15224 | 1 | [0,3,6,14,21,22,28,48,49,59,77,118... | |
| 1 | -15224 | 1 | [0,2,6,13,20,21,27,57,58,65,83,130,... | |
| 2 | -15224 | 1 | [0,2,5,19,20,23,30,62,63,67,85,138,... | |
| 3 | -15224 | 1 | [0,2,3,8,9,13,25,32,36,37,70,92,93,... | |
| 4 | -15224 | 1 | [0,1,5,10,11,17,28,34,40,41,72,96,... | |
| 5 | -15224 | 1 | [0,1,5,8,11,19,26,32,36,43,70,92,9,... | |
| 6 | -15224 | 1 | [0,1,4,9,10,15,27,39,48,49,77,104,... | |

# FIG. 24D

$\lambda_{\text{CONNECT}}=20, \lambda_{\text{ONE}}=20, \lambda_{\text{OVERLAP}}=20$

| | ENERGY | FREQUENCY | | BIT |
|---|---|---|---|---|
| 0 | -20224 | 1 | [0,4,6,17,22,23,29,61,62,67,85,136,... | |
| 1 | -20224 | 1 | [0,4,6,15,21,22,27,58,59,65,83,132,... | |
| 2 | -20224 | 1 | [0,4,5,17,22,23,30,60,61,66,84,135,... | |
| 3 | -20224 | 1 | [0,4,5,10,11,17,25,34,40,41,72,96,... | |
| 4 | -20224 | 2 | [0,2,5,19,20,23,30,56,63,64,82,139,... | |
| 5 | -20224 | 1 | [0,2,5,8,11,19,25,35,42,43,73,98,9,... | |
| 6 | -20224 | 1 | [0,1,4,10,11,17,29,41,51,52,79,107,... | |

# FIG. 24E

$\lambda_{\text{CONNECT}}=25, \lambda_{\text{ONE}}=25, \lambda_{\text{OVERLAP}}=25$

| | ENERGY | FREQUENCY | | BIT |
|---|---|---|---|---|
| 0 | -25224 | 2 | [0,4,5,17,22,23,30,60,61,66,84,135... | |
| 1 | -25224 | 1 | [0,4,5,10,11,17,25,35,41,42,73,97,... | |
| 2 | -25224 | 1 | [0,4,5,10,11,17,25,34,40,41,72,96,... | |
| 3 | -25224 | 1 | [0,2,6,19,20,23,29,62,63,67,85,138,... | |
| 4 | -25224 | 1 | [0,2,6,19,20,23,29,54,55,63,81,126,... | |
| 5 | -25224 | 1 | [0,2,3,13,20,21,30,57,58,65,83,130,... | |
| 6 | -25213 | 1 | [0,5,6,17,22,23,28,51,60,61,78,79,... | |

# FIG. 24F

$\lambda_{\text{CONNECT}}=30, \lambda_{\text{ONE}}=30, \lambda_{\text{OVERLAP}}=30$

| | ENERGY | FREQUENCY | | BIT |
|---|---|---|---|---|
| 0 | -30224 | 1 | [0,3,6.13,20,21,26,45,46,57,75,114... | |
| 1 | -30224 | 2 | [0,3,4,9,10,15,25,34,39,40,72,95,9... | |
| 2 | -30224 | 1 | [0,2,5,8,11,19,25,32,36,43,70,92,9... | |
| 3 | -30213 | 2 | [0,4,6,15,21,22,27,48,58,59,76,77,... | |
| 4 | -30213 | 1 | [0,4,5,10,11,17,25,34,35,41,69,72,... | |
| 5 | -30213 | 1 | [0,2,6,19,20,23,29,54,62,63,80,81,... | |
| 6 | -30213 | 1 | [0,2,5,19,20,23,30,63,64,67,82,85,... | |

# FIG. 25A

$\lambda_{CONNECT}=5, \lambda_{ONE}=30, \lambda_{OVERLAP}=30$

| | ENERGY | FREQUENCY | | BIT |
|---|---|---|---|---|
| 0 | -56 | 2 | [6,24,68,148,192,377,523,611,757] | |
| 1 | -56 | 1 | [0,6,24,68,151,195,377,523,608,747] | |
| 2 | -53 | 1 | [3,15,39,95,162,302,383,531,621] | |
| 3 | -49 | 1 | [6,24,68,152,297,381,523,608,754] | |
| 4 | -49 | 1 | [6,24,68,152,297,381,521,610,756] | |
| 5 | -49 | 3 | [6,24,68,152,296,380,521,611,757] | |
| 6 | -49 | 1 | [6,24,68,151,291,380,526,612,757] | |

# FIG. 25B

$\lambda_{CONNECT}=10, \lambda_{ONE}=30, \lambda_{OVERLAP}=30$

| | ENERGY | FREQUENCY | | BIT |
|---|---|---|---|---|
| 0 | -177 | 1 | [0,5,11,25,72,169,327,393,529,620] | |
| 1 | -177 | 1 | [0,4,22,30,85,189,375,424,546,652] | |
| 2 | -177 | 1 | [0,3,9,25,70,165,323,391,529,622] | |
| 3 | -99 | 1 | [0,5,17,28,78,179,337,399,533,613] | |
| 4 | -89 | 1 | [0,5,23,30,82,191,377,425,611,757] | |
| 5 | -89 | 1 | [0,3,21,30,84,187,373,423,609,748] | |
| 6 | -89 | 1 | [0,2,20,30,85,191,377,422,608,656] | |

# FIG. 25C

$\lambda_{CONNECT}=15, \lambda_{ONE}=30, \lambda_{OVERLAP}=30$

| | ENERGY | FREQUENCY | | BIT |
|---|---|---|---|---|
| 0 | -138 | 1 | [0,4,10,25,39,71,95,161,162,299,327,... | |
| 1 | -138 | 1 | [0,2,19,20,29,63,85,138,182,190,310,... | |
| 2 | -138 | 1 | [0,2,13,27,37,71,102,166,174,306,360,... | |
| 3 | -138 | 1 | [0,2,8,25,43,73,99,160,163,299,323,... | |
| 4 | -138 | 1 | [0,1,8,11,26,43,81,111,164,183,304,... | |
| 5 | -134 | 1 | [0,5,19,23,30,63,82,147,192,195,316,... | |
| 6 | -132 | 1 | [0,5,11,25,41,69,72,162,163,327,328,... | |

# FIG. 25D

$\lambda_{CONNECT}=20, \lambda_{ONE}=30, \lambda_{OVERLAP}=30$

| | ENERGY | FREQUENCY | | BIT |
|---|---|---|---|---|
| 0 | -7138 | 1 | [0,6,20,23,29,63,81,138,182,190,310,... | |
| 1 | -7138 | 1 | [0,5,17,23,30,61,84,145,194,195,316,... | |
| 2 | -7138 | 2 | [0,4,15,22,30,59,83,143,193,194,316,... | |
| 3 | -7138 | 1 | [0,4,15,22,27,59,83,132,176,186,306,... | |
| 4 | -7138 | 1 | [0,3,13,21,30,57,82,141,192,193,316,... | |
| 5 | -7138 | 1 | [0,3,9,13,26,37,70,101,164,173,304,... | |
| 6 | -7138 | 1 | [0,2,19,20,30,63,85,147,192,195,316,... | |

# FIG. 25E

$\lambda_{\text{CONNECT}}=25, \lambda_{\text{ONE}}=30, \lambda_{\text{OVERLAP}}=30$

| | ENERGY | FREQUENCY | | BIT |
|---|---|---|---|---|
| 0 | -16036 | | 1 [0,6,22,23,29,61,62,79,80,136,170,1... | |
| 1 | -16036 | | 1 [0,6,21,22,27,58,59,76,77,132,166,1... | |
| 2 | -16036 | | 2 [0,6,20,21,27,57,58,75,76,130,166,1... | |
| 3 | -16036 | | 1 [0,5,11,17,25,35,41,69,72,97,161,16... | |
| 4 | -16036 | | 1 [0,3,13,21,20,57,65,82,86,141,192,1... | |
| 5 | -16036 | | 1 [0,3,9,15,28,39,49,72,78,105,168,17... | |
| 6 | -16036 | | 2 [0,3,9,13,25,33,37,69,70,93,160,163... | |

# FIG. 25F

$\lambda_{\text{CONNECT}}=30, \lambda_{\text{ONE}}=30, \lambda_{\text{OVERLAP}}=30$

| | ENERGY | FREQUENCY | | BIT |
|---|---|---|---|---|
| 0 | -30224 | | 1 [0,3,6.13,20,21,26,45,46,57,75,114... | |
| 1 | -30224 | | 2 [0,3,4,9,10,15,25,34,39,40,72,95,9... | |
| 2 | -30224 | | 1 [0,2,5,8,11,19,25,32,36,43,70,92,9... | |
| 3 | -30213 | | 2 [0,4,6,15,21,22,27,48,58,59,76,77,... | |
| 4 | -30213 | | 1 [0,4,5,10,11,17,25,34,35,41,69,72,... | |
| 5 | -30213 | | 1 [0,2,6,19,20,23,29,54,62,63,80,81,... | |
| 6 | -30213 | | 1 [0,2,5,19,20,23,30,63,64,67,82,85,... | |

# FIG. 26A

$\lambda_{\text{CONNECT}}=5, \lambda_{\text{ONE}}=25, \lambda_{\text{OVERLAP}}=25$

| | ENERGY | FREQUENCY | | BIT |
|---|---|---|---|---|
| 0 | -56 | | 2 | [6,24,68,148,192,377,523,611,757] |
| 1 | -56 | | 1 | [0,6,24,68,151,195,377,523,608,747] |
| 2 | -53 | | 1 | [3,15,39,95,162,302,383,531,621] |
| 3 | -49 | | 1 | [6,24,68,152,297,381,523,608,754] |
| 4 | -49 | | 3 | [6,24,68,152,296,380,521,611,757] |
| 5 | -49 | | 1 | [6,24,68,152,294,378,523,611,757] |
| 6 | -49 | | 1 | [6,24,68,151,291,380,526,612,757] |

# FIG. 26B

$\lambda_{\text{CONNECT}}=10, \lambda_{\text{ONE}}=25, \lambda_{\text{OVERLAP}}=25$

| | ENERGY | FREQUENCY | | BIT |
|---|---|---|---|---|
| 0 | -123 | | 2 | [0,5,17,28,78,179,337,399,532,613] |
| 1 | -123 | | 1 | [0,5,11,25,69,163,329,401,530,614] |
| 2 | -123 | | 2 | [0,4,17,29,80,180,338,399,533,613] |
| 3 | -123 | | 1 | [0,4,15,27,76,176,334,397,531,613] |
| 4 | -123 | | 1 | [0,2,20,30,86,192,377,421,545,617] |
| 5 | -117 | | 1 | [0,6,23,29,81,191,368,420,540,640] |
| 6 | -117 | | 1 | [0,5,23,30,86,195,375,419,544,648] |

# FIG. 26C

$\lambda_{CONNECT}=15,\ \lambda_{ONE}=25,\ \lambda_{OVERLAP}=25$

| | ENERGY | FREQUENCY | BIT |
|---|---|---|---|
| 0 | -3638 | 1 | [0,6,20,23,26,63,81,139,184,304,... |
| 1 | -3638 | 1 | [0,6,20,21,26,57,75,129,173,184,304,... |
| 2 | -3638 | 1 | [0,5,11,17,25,41,72,97,162,163,299,... |
| 3 | -3638 | 1 | [0,4,17,22,29,61,85,136,180,190,310,... |
| 4 | -3638 | 1 | [0,4,15,22,30,59,83,143,193,194,316,... |
| 5 | -3638 | 1 | [0,4,10,17,25,41,73,97,162,163,299,... |
| 6 | -3638 | 1 | [0,3,15,21,30,59,84,143,193,194,316,... |

# FIG. 26D

$\lambda_{CONNECT}=20,\ \lambda_{ONE}=25,\ \lambda_{OVERLAP}=25$

| | ENERGY | FREQUENCY | BIT |
|---|---|---|---|
| 0 | -11114 | 1 | [0,5,17,23,30,61,84,145,194,195,316,... |
| 1 | -11114 | 1 | [0,4,17,22,30,61,85,145,194,195,316,... |
| 2 | -11114 | 1 | [0,4,17,22,29,61,85,136,180,190,310,... |
| 3 | -11114 | 1 | [0,4,10,17,29,61,85,136,180,190,310,... |
| 4 | -11114 | 1 | [0,3,15,21,30,59,84,143,193,194,316,... |
| 5 | -11114 | 1 | [0,3,15,21,28,59,84,133,177,188,308,... |
| 6 | -11114 | 1 | [0,3,13,21,30,57,84,141,192,193,316,... |

# FIG. 26E

$\lambda_{CONNECT}=25, \lambda_{ONE}=25, \lambda_{OVERLAP}=25$

| | ENERGY | FREQUENCY | BIT |
|---|---|---|---|
| 0 | -25224 | 2 | [0,4,5,17,22,23,30,60,61,66,84,135,... |
| 1 | -25224 | 1 | [0,4,5,10,11,17,25,35,41,42,73,97,... |
| 2 | -25224 | 1 | [0,4,5,10,11,17,25,34,40,41,72,96,... |
| 3 | -25224 | 1 | [0,2,6,19,20,23,29,62,63,67,85,138,... |
| 4 | -25224 | 1 | [0,2,6,19,20,23,29,54,55,63,81,126,... |
| 5 | -25224 | 1 | [0,2,3,13,20,21,30,57,58,65,83,130,... |
| 6 | -25213 | 1 | [0,5,6,17,22,23,28,51,60,61,78,79,... |

# FIG. 27A

$\lambda_{CONNECT}=5, \lambda_{ONE}=5, \lambda_{OVERLAP}=5$

| | ENERGY | FREQUENCY | | BIT |
|---|---|---|---|---|
| 0 | -123 | | 2 | [0,6,23,28,75,251,367,482,536,613] |
| 1 | -123 | | 1 | [0,6,23,28,75,251,365,480,534,615] |
| 2 | -123 | | 2 | [0,6,20,25,69,245,368,482,536,616] |
| 3 | -123 | | 1 | [0,6,20,25,69,245,358,476,530,614] |
| 4 | -123 | | 3 | [0,4,23,55,139,272,377,482,583,613] |
| 5 | -123 | | 5 | [0,4,23,55,139,272,375,480,585,615] |
| 6 | -123 | | 1 | [0,3,22,55,139,271,375,480,586,616] |

# FIG. 27B

$\lambda_{CONNECT}=10, \lambda_{ONE}=10, \lambda_{OVERLAP}=10$

| | ENERGY | FREQUENCY | | BIT |
|---|---|---|---|---|
| 0 | -123 | | 1 | [0,6,23,28,75,251,367,482,536,613] |
| 1 | -123 | | 3 | [0,6,20,25,69,245,368,482,536,616] |
| 2 | -123 | | 1 | [0,5,18,28,75,196,335,425,534,615] |
| 3 | -123 | | 1 | [0,4,23,55,139,272,377,482,583,613] |
| 4 | -123 | | 2 | [0,4,23,55,139,272,375,480,585,615] |
| 5 | -123 | | 1 | [0,3,22,55,139,271,375,480,586,616] |
| 6 | -123 | | 1 | [0,3,22,55,139,271,373,478,584,614] |

# FIG. 27C

$\lambda_{\text{CONNECT}}=15, \lambda_{\text{ONE}}=15, \lambda_{\text{OVERLAP}}=15$

| | ENERGY | FREQUENCY | | BIT |
|---|---|---|---|---|
| 0 | -123 | | 1 | [0,6,23,28,75,251,367,482,536,613] |
| 1 | -123 | | 4 | [0,6,23,28,75,251,365,480,534,615] |
| 2 | -123 | | 1 | [0,6,21,26,71,247,361,478,532,615] |
| 3 | -123 | | 2 | [0,4,23,55,139,272,377,482,583,613] |
| 4 | -123 | | 5 | [0,4,23,55,139,272,375,482,583,615] |
| 5 | -123 | | 1 | [0,3,22,55,139,271,373,478,584,614] |
| 6 | -123 | | 1 | [0,3,17,41,109,216,345,425,556,616] |

# FIG. 27D

$\lambda_{\text{CONNECT}}=20, \lambda_{\text{ONE}}=20, \lambda_{\text{OVERLAP}}=20$

| | ENERGY | FREQUENCY | | BIT |
|---|---|---|---|---|
| 0 | -123 | | 1 | [0,6,23,28,75,251,367,482,536,613] |
| 1 | -123 | | 1 | [0,6,22,27,73,249,362,478,532,614] |
| 2 | -123 | | 1 | [0,6,21,26,71,247,361,478,532,615] |
| 3 | -123 | | 1 | [0,6,21,26,71,247,361,478,532,614] |
| 4 | -123 | | 1 | [0,4,23,55,139,272,377,482,583,613] |
| 5 | -123 | | 6 | [0,4,23,55,139,272,375,480,585,615] |
| 6 | -123 | | 1 | [0,3,22,55,139,271,373,478,584,614] |

# FIG. 27E

$\lambda_{CONNECT}=25, \lambda_{ONE}=25, \lambda_{OVERLAP}=25$

| | ENERGY | FREQUENCY | | BIT |
|---|---|---|---|---|
| 0 | -123 | | 2 | [0,6,23,28,75,251,365,480,534,615] |
| 1 | -123 | | 1 | [0,6,21,26,71,247,359,476,530,613] |
| 2 | -123 | | 2 | [0,4,23,55,139,272,377,482,583,613] |
| 3 | -123 | | 2 | [0,4,23,55,139,272,375,480,585,615] |
| 4 | -123 | | 1 | [0,3,22,55,139,271,375,480,586,616] |
| 5 | -123 | | 1 | [0,3,22,55,139,271,373,478,584,614] |
| 6 | -123 | | 1 | [0,2,21,55,139,270,373,478,585,615] |

# FIG. 27F

$\lambda_{CONNECT}=30, \lambda_{ONE}=30, \lambda_{OVERLAP}=30$

| | ENERGY | FREQUENCY | | BIT |
|---|---|---|---|---|
| 0 | -123 | | 1 | [0,6,23,28,75,251,367,482,536,613] |
| 1 | -123 | | 1 | [0,6,22,27,73,249,362,478,532,614] |
| 2 | -123 | | 2 | [0,6,21,26,71,247,361,478,532,615] |
| 3 | -123 | | 1 | [0,6,20,25,69,245,358,476,530,614] |
| 4 | -123 | | 1 | [0,4,23,55,139,272,377,482,583,613] |
| 5 | -123 | | 6 | [0,4,23,55,139,272,375,480,585,615] |
| 6 | -123 | | 1 | [0,3,22,55,139,271,375,480,586,616] |

# FIG. 28A

| | ENERGY | FREQ | PENALTY | | BIT |
|---|---|---|---|---|---|
| 0 | -436 | 1 | [0] | [0,1,5,19,123,335,504,521,539,547,567,589,1125,1438,1658,1718] | |
| 1 | -432 | 10 | [0] | [0,1,2,87,300,456,513,531,541,553,561,597,1148,1541,1645,1749] | |
| 2 | -432 | 8 | [0] | [0,1,2,87,300,436,513,531,541,553,561,597,1204,1449,1645,1749] | |
| 3 | -423 | 11 | [0] | [0,1,5,42,110,411,513,531,541,553,561,597,834,1471,1645,1749] | |
| 4 | -422 | 4 | [0] | [0,1,5,67,203,383,483,521,535,553,568,705,894,1373,1597,1739] | |
| 5 | -420 | 2 | [0] | [0,1,2,87,300,456,513,531,541,553,561,597,1212,1503,1645,1749] | |
| 6 | -420 | 8 | [0] | [0,1,2,87,292,452,512,531,541,553,561,597,1204,1535,1645,1749] | |
| 7 | -419 | 1 | [0] | [0,1,3,89,303,439,507,522,542,547,578,599,1229,1469,1646,1725] | |
| 8 | -419 | 2 | [0] | [0,1,5,55,177,371,483,521,539,549,567,705,871,1303,1584,1750] | |
| 9 | -419 | 2 | [0] | [0,1,2,87,300,436,513,531,541,553,561,597,1225,1526,1645,1749] | |
| 10 | -419 | 2 | [0] | [0,1,3,88,301,437,504,521,541,546,579,597,1226,1467,1645,1722] | |
| 11 | -419 | 1 | [0] | [0,1,3,43,192,376,483,521,537,550,566,597,1012,1333,1584,1743] | |
| 12 | -418 | 3 | [0] | [0,1,5,55,214,394,495,527,537,552,575,629,1038,1379,1631,1718] | |
| 13 | -418 | 2 | [0] | [0,1,5,55,214,371,483,521,535,553,568,629,1044,1337,1597,1739] | |
| 14 | -418 | 1 | [0] | [0,1,2,87,300,436,513,531,541,553,561,597,1212,1437,1645,1749] | |
| 15 | -418 | 1 | [0] | [0,1,5,42,191,375,492,527,537,550,566,597,998,1303,1584,1728] | |
| 16 | -416 | 1 | [0] | [0,1,5,55,214,394,495,527,537,550,567,670,1038,1379,1631,1718] | |
| 17 | -414 | 3 | [0] | [0,1,2,33,101,345,478,523,542,547,448,669,1106,1314,1667,1721] | |
| 18 | -414 | 1 | [0] | [0,1,2,87,292,452,512,531,541,553,561,597,1144,1537,1645,1749] | |
| 19 | -414 | 6 | [0] | [0,1,2,78,101,345,478,523,538,551,558,778,892,1448,1606,1742] | |
| 20 | -414 | 1 | [0] | [0,1,2,87,292,452,512,531,541,553,561,597,1144,1535,1645,1749] | |

# FIG. 28B

# FIG. 29A

| | ENERGY | FREQ | PENALTY | BIT |
|---|---|---|---|---|
| 0 | -436 | 67 | [0] | [0,1,5,19,123,335,504,521,539,547,567,589,1125,1438,1658,1718] |
| 1 | -432 | 19 | [0] | [0,1,2,87,300,456,513,531,541,553,561,597,1148,1541,1645,1749] |
| 2 | -432 | 7 | [0] | [0,1,2,87,300,436,513,531,541,553,561,597,1204,1449,1645,1749] |
| 3 | -432 | 2 | [0] | [0,1,2,42,191,375,492,527,537,549,576,597,990,1315,1584,1728] |
| 4 | -432 | 2 | [0] | [0,1,2,42,191,395,492,527,537,549,576,597,934,1407,1584,1728] |
| 5 | -432 | 2 | [0] | [0,1,5,42,191,375,492,527,537,550,566,597,990,1315,1584,1728] |
| 6 | -432 | 1 | [0] | [0,1,5,42,191,395,492,527,537,550,566,597,934,1407,1584,1728] |

# FIG. 29B

|  |  |
|---|---|
| ENERGY: | -436 |
| FREQENCY: | 67 |
| RMSD: | 0.91-1.42 |

-432
19
0.80-1.63

-432
7
0.64-1.52

-432
2
1.73-2.10

|  |  |
|---|---|
| ENERGY: | -432 |
| FREQENCY: | 2 |
| RMSD: | 1.66-2.99 |

-432
2
1.24-1.63

-432
1
1.20-2.02

# FIG. 29C

Europäisches
Patentamt

European
Patent Office

Office européen
des brevets

# EUROPEAN SEARCH REPORT

Application Number

EP 21 18 8468

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 667 673 A1 (FUJITSU LTD [JP]) 17 June 2020 (2020-06-17) * the whole document * | 1-15 | INV. G16C20/40 G16B15/00 |
| X,D | Ryan Babbush ET AL: "Construction of Energy Functions for Lattice Heteropolymer Models: Efficient Encodings for Constraint Satisfaction Programming and Quantum Annealing" In: "Advances in Chemical Physics: Volume 155", 11 April 2014 (2014-04-11), John Wiley & Sons, Inc., Hoboken, New Jersey, XP055692281, ISBN: 978-1-118-75577-8 pages 201-244, DOI: 10.1002/9781118755815.ch05, * the whole document * | 1-15 | |
| X | TOMAS BABEJ ET AL: "Coarse-grained lattice protein folding on a quantum annealer", ARXIV.ORG, CORNELL UNIVERSITY LIBRARY, 201 OLIN LIBRARY CORNELL UNIVERSITY ITHACA, NY 14853, 2 November 2018 (2018-11-02), XP080941413, * the whole document * | 1-15 | TECHNICAL FIELDS SEARCHED (IPC)  G16C G16B |
| X | MARK FINGERHUTH ET AL: "A quantum alternating operator ansatz with hard and soft constraints for lattice protein folding", ARXIV.ORG, CORNELL UNIVERSITY LIBRARY, 201 OLIN LIBRARY CORNELL UNIVERSITY ITHACA, NY 14853, 31 October 2018 (2018-10-31), XP080933694, * the whole document * | 1-15 | |

The present search report has been drawn up for all claims

| Place of search | Date of completion of the search | Examiner |
|---|---|---|
| The Hague | 14 January 2022 | Denoual, Matthieu |

## ANNEX TO THE EUROPEAN SEARCH REPORT
## ON EUROPEAN PATENT APPLICATION NO.

EP 21 18 8468

This annex lists the patent family members relating to the patent documents cited in the above-mentioned European search report.
The members are as contained in the European Patent Office EDP file on
The European Patent Office is in no way liable for these particulars which are merely given for the purpose of information.

14-01-2022

| Patent document cited in search report | Publication date | Patent family member(s) | Publication date |
|---|---|---|---|
| EP 3667673 A1 | 17-06-2020 | CN 111261235 A<br>EP 3667673 A1<br>JP 2020091518 A<br>US 2020176074 A1 | 09-06-2020<br>17-06-2020<br>11-06-2020<br>04-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. BABBUSH et al.** Construction of Energy Functions for Lattice Heteropolymer Models: A Case Study in Constraint Satisfaction Programming and Adiabatic Quantum Optimization. *Advances in Chemical Physics,* vol. 155, 201-244